(19) Europäisches Patentamt
European Patent Office
Office européen des brevets

(11) EP 2 163 650 B1

(12) **EUROPEAN PATENT SPECIFICATION**

(45) Date of publication and mention of the grant of the patent:
**05.08.2015 Bulletin 2015/32**

(51) Int Cl.:
***C12Q 1/68*** *(2006.01)*

(21) Application number: **09014283.7**

(22) Date of filing: **07.04.2005**

(54) **Gene expression markers for predicting response to chemotherapy**

Genexpressionsmarker zur Vorhersage des Erfolgs von Chemotherapy

Marqueurs d'expression de gènes pour prédire une réponse à la chimiothérapie

(84) Designated Contracting States:
**AT BE BG CH CY CZ DE DK EE ES FI FR GB GR HU IE IS IT LI LT LU MC NL PL PT RO SE SI SK TR**

(30) Priority: **09.04.2004 US 561035 P**

(43) Date of publication of application:
**17.03.2010 Bulletin 2010/11**

(60) Divisional application:
**10177408.1 / 2 270 232**
**15173816.8**

(62) Document number(s) of the earlier application(s) in accordance with Art. 76 EPC:
**05735478.9 / 1 737 980**

(73) Proprietors:
- **Genomic Health, Inc.**
  **Redwood City, CA 94063 (US)**
- **Fondazione IRCCS Istituto Nazionale dei Tumori**
  **20133 Milan (IT)**

(72) Inventors:
- **Baker, Joffre, B.**
  **Montara, CA 94037 (US)**
- **Shak, Steven**
  **Hillsborough, CA 94010 (US)**
- **Gianni, Luca**
  **20133 Milano (IT)**

(74) Representative: **Tollett, Ian et al**
**Williams Powell**
**Staple Court**
**11 Staple Inn Buildings**
**London, WC1V 7QH (GB)**

(56) References cited:
**WO-A2-2004/111603**

- **DING Z ET AL: "Multidrug resistance in human cervical cells is associated with enhanced expression of anti-apoptotic proteins BAG-1 and Bcl-XL and reduced caspase-3 activity" PROCEEDINGS OF THE AMERICAN ASSOCIATION FOR CANCER RESEARCH ANNUAL MEETING, no. 41, March 2000 (2000-03), page 404, XP001537327 & 91ST ANNUAL MEETING OF THE AMERICAN ASSOCIATION FOR CANCER RESEARCH.; SAN FRANCISCO, CALIFORNIA, USA; APRIL 01-05, 2000 ISSN: 0197-016X**
- **KITADA SHINICHI ET AL: "Expression of apoptosis-regulating proteins in chronic lymphocytic leukemia: Correlations with in vitro and in vivo chemoresponses" BLOOD, vol. 91, no. 9, 1 May 1998 (1998-05-01), pages 3379-3389, XP002566452 ISSN: 0006-4971**
- **PUSZTAI LAJOS ET AL: "Expression of BAG-1 and BcL-2 proteins before and after neoadjuvant chemotherapy of locally advanced breast cancer" CANCER INVESTIGATION, vol. 22, no. 2, 2004, pages 248-256, XP008118205 ISSN: 0735-7907**
- **MUSS H B ET AL: "C-ERBB-2 EXPRESSION AND RESPONSE TO ADJUVANT THERAPY IN WOMEN WITH NODE-POSITIVE EARLY BREAST CANCER" NEW ENGLAND JOURNAL OF MEDICINE, MASSACHUSETTS MEDICAL SOCIETY, BOSTON, MA, US, vol. 330, no. 18, 5 May 1994 (1994-05-05), pages 1260-1266, XP009040793 ISSN: 1533-4406**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- RING A E ET AL: "PREDICTORS OF RESPONSE TO SYSTEMIC THERAPY IN BREAST CANCER" FORUM, GENOVA, IT, vol. 12, no. 1, 2002, pages 19-32, XP009012703 ISSN: 1121-8142
- BERTUCCI F ET AL: "GENE EXPRESSION PROFILES OF POOR-PROGNOSIS PRIMARY BREAST CANCER CORRELATE WITH SURVIVAL" HUMAN MOLECULAR GENETICS, OXFORD UNIVERSITY PRESS, SURREY, GB, vol. 11, no. 8, 15 April 2002 (2002-04-15) , pages 863-872, XP001146244 ISSN: 0964-6906
- STEARNS V ET AL: "A prospective randomized pilot study to evaluate predictors of response in serial core biopsies to single agent neoadjuvant doxorubicin or paclitaxel for patients with locally advanced breast cancer" CLINICAL CANCER RESEARCH, THE AMERICAN ASSOCIATION FOR CANCER RESEARCH, US, vol. 9, no. 1, January 2003 (2003-01), pages 124-133, XP002318509 ISSN: 1078-0432
- SJÖSTRÖM JOHANNA ET AL: "The predictive value of bcl-2, bax, bcl-xL, bag-1, fas, and fasL for chemotherapy response in advanced breast cancer.", CLINICAL CANCER RESEARCH : AN OFFICIAL JOURNAL OF THE AMERICAN ASSOCIATION FOR CANCER RESEARCH MAR 2002, vol. 8, no. 3, March 2002 (2002-03), pages 811-816, ISSN: 1078-0432

SEQUENCE LISTING

<110> GENOMIC HEALTH, INC.
BAKER, JOFFRE B.
SHAK, STEVEN
GIANNI, LUCA

<120> GENE EXPRESSION MARKERS FOR PREDICTING RESPONSE TO CHEMOTHERAPY

<130> H64514PCEPT1

<140> Divisional of 05735478.9
<141> 2005-04-07

<160> 340

<170> FastSEQ for windows version 4.0

<210> 1
<211> 23
<212> DNA
<213> Artificial sequence

<220>
<223> Synthetic Oligonucleotide Forward Primer

<400> 1
cgttccgatc ctctatactg cat 23

<210> 2
<211> 22
<212> DNA
<213> Artificial Sequence

<220>
<223> synthetic oligonucleotide Reverse Primer

<400> 2
aggtccctgt tggccttata gg 22

<210> 3
<211> 25
<212> DNA
<213> Artificial Sequence

<220>
<223> Synthetic Oligonucleotide Probe

<400> 3
atgcctacag caccctgatg tcgca 25

<210> 4
<211> 20
<212> DNA
<213> Artificial sequence

<220>
<223> synthetic oligonucleotide Forward Primer

<400> 4
tgcagactgt accatgctga 20

<210> 5
<211> 20
<212> DNA
<213> Artificial sequence

<220>
<223> Synthetic Oligonucleotide Reverse Primer

<400> 5
ggccagcacc ataatcctat 20

<210> 6
<211> 22
<212> DNA
<213> Artificial Sequence

<220>
<223> Synthetic Oligonucleotide Probe

<400> 6
ctgcacacgg ttctaggctc cg 22

<210> 7
<211> 27
<212> DNA
<213> Artificial Sequence

<220>
<223> Synthetic oligonucleotide Forward Primer

<400> 7
ctgcatgtga ttgaataaga aacaaga 27

<210> 8
<211> 22
<212> DNA
<213> Artificial Sequence

<220>
<223> Synthetic Oligonucleotide Reverse Primer

<400> 8
tgtggacctg atccctgtac ac 22

<210> 9
<211> 23
<212> DNA
<213> Artificial sequence

<220>
<223> Synthetic oligonucleotide Probe

<400> 9
tgaccacacc aaagcctccc tgg 23

<210> 10
<211> 20
<212> DNA
<213> Artificial Sequence

<220>
<223> Synthetic oligonucleotide Forward Primer

<400> 10
acgaattgtc ggtgaggtct 20

<210> 11
<211> 20
<212> DNA
<213> Artificial Sequence

<220>
<223> Synthetic oligonucleotide Reverse Primer

<400> 11
gtccatgctg aaatcattgg 20

<210> 12
<211> 25
<212> DNA
<213> Artificial sequence

<220>
<223> Synthetic oligonucleotide Probe

<400> 12
caggatacca cagtcctgga gaccc 25

<210> 13
<211> 20
<212> DNA
<213> Artificial Sequence

<220>
<223> Synthetic oligonucleotide Forward Primer

<400> 13
cgcttctatg gcgctgagat 20

<210> 14
<211> 20
<212> DNA
<213> Artificial Sequence

<220>
<223> Synthetic oligonucleotide Reverse Primer

<400> 14
tcccggtaca ccacgttctt 20

<210> 15
<211> 24
<212> DNA
<213> Artificial Sequence

<220>
<223> Synthetic Oligonucleotide Probe

<400> 15
cagccctgga ctacctgcac tcgg 24

<210> 16
<211> 19
<212> DNA
<213> Artificial Sequence

<220>
<223> Synthetic oligonucleotide Forward Primer

<400> 16
tcctgccacc cttcaaacc 19

<210> 17
<211> 21
<212> DNA
<213> Artificial Sequence

<220>
<223> Synthetic oligonucleotide Reverse Primer

<400> 17
ggcggtaaat tcatcatcga a 21

<210> 18
<211> 24
<212> DNA
<213> Artificial sequence

<220>
<223> Synthetic Oligonucleotide Probe

<400> 18
caggtcacgt ccgaggtcga caca 24

<210> 19
<211> 20
<212> DNA
<213> Artificial sequence

<220>
<223> Synthetic oligonucleotide Forward Primer

<400> 19
ggacagcagg aatgtgtttc 20

<210> 20
<211> 20
<212> DNA

<213> Artificial Sequence

<220>
<223> Synthetic oligonucleotide Reverse Primer

<400> 20
acccactcga tttgtttctg 20

<210> 21
<211> 22
<212> DNA
<213> Artificial sequence

<220>
<223> Synthetic oligonucleotide Probe

<400> 21
cattggctcc ccgtgacctg ta 22

<210> 22
<211> 19
<212> DNA
<213> Artificial Sequence

<220>
<223> Synthetic oligonucleotide Forward Primer

<400> 22
gggtcaggtg cctcgagat 19

<210> 23
<211> 21
<212> DNA
<213> Artificial Sequence

<220>
<223> Synthetic oligonucleotide Reverse Primer

<400> 23
ctgctcactc ggctcaaact c 21

<210> 24
<211> 24
<212> DNA
<213> Artificial Sequence

<220>
<223> Synthetic oligonucleotide Probe

<400> 24
tgggcccaga gcatgttcca gatc 24

<210> 25
<211> 23
<212> DNA
<213> Artificial Sequence

<220>

<223> Synthetic oligonucleotide Forward Primer

<400> 25
cgttgtcagc acttggaata caa 23

<210> 26
<211> 24
<212> DNA
<213> Artificial Sequence

<220>
<223> Synthetic Oligonucleotide Reverse Primer

<400> 26
gttcaacctc ttcctgtgga ctgt 24

<210> 27
<211> 26
<212> DNA
<213> Artificial Sequence

<220>
<223> Synthetic oligonucleotide Probe

<400> 27
cccaattaac atgacccggc aaccat 26

<210> 28
<211> 20
<212> DNA
<213> Artificial Sequence

<220>
<223> Synthetic Oligonucleotide Forward Primer

<400> 28
cctggagggt cctgtacaat 20

<210> 29
<211> 19
<212> DNA
<213> Artificial sequence

<220>
<223> Synthetic oligonucleotide Reverse Primer

<400> 29
ctaattgggc tccatctcg 19

<210> 30
<211> 24
<212> DNA
<213> Artificial Sequence

<220>
<223> Synthetic oligonucleotide Probe

<400> 30

catcatggga ctcctgccct tacc 24

<210> 31
<211> 25
<212> DNA
<213> Artificial Sequence

<220>
<223> Synthetic oligonucleotide Forward Primer

<400> 31
cagatggacc tagtacccac tgaga 25

<210> 32
<211> 24
<212> DNA
<213> Artificial Sequence

<220>
<223> Synthetic oligonucleotide Reverse Primer

<400> 32
cctatgattt aagggcattt ttcc 24

<210> 33
<211> 22
<212> DNA
<213> Artificial Sequence

<220>
<223> Synthetic oligonucleotide Probe

<400> 33
ttccacgccg aaggacagcg at 22

<210> 34
<211> 21
<212> DNA
<213> Artificial Sequence

<220>
<223> Synthetic Oligonucleotide Forward Primer

<400> 34
gcatgttcgt ggcctctaag a 21

<210> 35
<211> 22
<212> DNA
<213> Artificial sequence

<220>
<223> Synthetic oligonucleotide Reverse Primer

<400> 35
cggtgtagat gcacagcttc tc 22

<210> 36

<211> 23
<212> DNA
<213> Artificial Sequence

<220>
<223> synthetic oligonucleotide Probe

<400> 36
aaggagacca tccccctgac ggc 23

<210> 37
<211> 20
<212> DNA
<213> Artificial Sequence

<220>
<223> Synthetic oligonucleotide Forward Primer

<400> 37
cgtcaggacc caccatgtct 20

<210> 38
<211> 24
<212> DNA
<213> Artificial Sequence

<220>
<223> Synthetic oligonucleotide Reverse Primer

<400> 38
ggttaattgg tgacatcctc aaga 24

<210> 39
<211> 20
<212> DNA
<213> Artificial Sequence

<220>
<223> Synthetic oligonucleotide Probe

<400> 39
cgcggccgag acatggcttg 20

<210> 40
<211> 25
<212> DNA
<213> Artificial Sequence

<220>
<223> Synthetic oligonucleotide Forward Primer

<400> 40
tgtatttcaa gacctctgtg cactt 25

<210> 41
<211> 23
<212> DNA
<213> Artificial Sequence

<220>
<223> Synthetic oligonucleotide Reverse Primer

<400> 41
ttagcctgag gaattgctgt gtt 23

<210> 42
<211> 25
<212> DNA
<213> Artificial Sequence

<220>
<223> Synthetic oligonucleotide Probe

<400> 42
tttatgaacc tgccctgctc ccaca 25

<210> 43
<211> 20
<212> DNA
<213> Artificial Sequence

<220>
<223> Synthetic oligonucleotide Forward Primer

<400> 43
agatgaagtg gaaggcgctt 20

<210> 44
<211> 21
<212> DNA
<213> Artificial Sequence

<220>
<223> Synthetic oligonucleotide Reverse Primer

<400> 44
tgcctctgta atcggcaact g 21

<210> 45
<211> 18
<212> DNA
<213> Artificial Sequence

<220>
<223> Synthetic oligonucleotide Probe

<400> 45
caccgcggcc atcctgca 18

<210> 46
<211> 20
<212> DNA
<213> Artificial Sequence

<220>
<223> Synthetic oligonucleotide Forward Primer

<400> 46
gggcgtggaa cagtttatct 20

<210> 47
<211> 19
<212> DNA
<213> Artificial Sequence

<220>
<223> Synthetic oligonucleotide Reverse Primer

<400> 47
cacggtgaag gtttcgagt 19

<210> 48
<211> 24
<212> DNA
<213> Artificial Sequence

<220>
<223> Synthetic oligonucleotide Probe

<400> 48
agacatctgc cccaagaagg acgt 24

<210> 49
<211> 21
<212> DNA
<213> Artificial sequence

<220>
<223> Synthetic oligonucleotide Forward Primer

<400> 49
tggattggag ttctgggaat g 21

<210> 50
<211> 22
<212> DNA
<213> Artificial Sequence

<220>
<223> Synthetic oligonucleotide Reverse Primer

<400> 50
gcttgcactc cacaggtaca ca 22

<210> 51
<211> 23
<212> DNA
<213> Artificial Sequence

<220>
<223> Synthetic oligonucleotide Probe

<400> 51
actggccgtg gcactggaca aca 23

<210> 52
<211> 21
<212> DNA
<213> Artificial sequence

<220>
<223> Synthetic oligonucleotide Forward Primer

<400> 52
aaacgagcag tttgccatca g 21

<210> 53
<211> 20
<212> DNA
<213> Artificial Sequence

<220>
<223> synthetic Oligonucleotide Reverse Primer

<400> 53
gttggtgatg ttccgaagca 20

<210> 54
<211> 24
<212> DNA
<213> Artificial Sequence

<220>
<223> Synthetic oligonucleotide Probe

<400> 54
cctcaccggc atagactgga agcg 24

<210> 55
<211> 21
<212> DNA
<213> Artificial Sequence

<220>
<223> Synthetic oligonucleotide Forward Primer

<400> 55
tgacaatcag cacacctgca t 21

<210> 56
<211> 23
<212> DNA
<213> Artificial Sequence

<220>
<223> synthetic oligonucleotide Reverse Primer

<400> 56
tgtgactaca gccgtgatcc tta 23

<210> 57
<211> 20
<212> DNA

<213> Artificial Sequence

<220>
<223> Synthetic oligonucleotide Probe

<400> 57
caggccctct tccgagcggt 20

<210> 58
<211> 20
<212> DNA
<213> Artificial Sequence

<220>
<223> synthetic oligonucleotide Forward Primer

<400> 58
ctgaaggagc tccaagacct 20

<210> 59
<211> 20
<212> DNA
<213> Artificial Sequence

<220>
<223> Synthetic Oligonucleotide Reverse Primer

<400> 59
caaaaccgct gtgtttcttc 20

<210> 60
<211> 22
<212> DNA
<213> Artificial Sequence

<220>
<223> Synthetic oligonucleotide Probe

<400> 60
tgctgatgtg ccctctcctt gg 22

<210> 61
<211> 18
<212> DNA
<213> Artificial sequence

<220>
<223> Synthetic oligonucleotide Forward Primer

<400> 61
gtggccatcc agctgacc 18

<210> 62
<211> 23
<212> DNA
<213> Artificial Sequence

<220>

<223> Synthetic Oligonucleotide Reverse Primer

<400> 62
cagtggtagg tgatgttctg gga 23

<210> 63
<211> 21
<212> DNA
<213> Artificial sequence

<220>
<223> Synthetic oligonucleotide Probe

<400> 63
tcctgcgcct gatgtccacc g 21

<210> 64
<211> 24
<212> DNA
<213> Artificial sequence

<220>
<223> Synthetic oligonucleotide Forward Primer

<400> 64
cagccaagaa ctggtatagg agct 24

<210> 65
<211> 19
<212> DNA
<213> Artificial Sequence

<220>
<223> Synthetic oligonucleotide Reverse Primer

<400> 65
aaactggctg ccagcattg 19

<210> 66
<211> 30
<212> DNA
<213> Artificial sequence

<220>
<223> Synthetic oligonucleotide Probe

<400> 66
tctcctagcc agacgtgttt cttgtccttg 30

<210> 67
<211> 24
<212> DNA
<213> Artificial Sequence

<220>
<223> Synthetic oligonucleotide Forward Primer

<400> 67

cgacagttgc gatgaaagtt ctaa 24

<210> 68
<211> 22
<212> DNA
<213> Artificial sequence

<220>
<223> Synthetic oligonucleotide Reverse Primer

<400> 68
ggctgctaga gaccatggac at 22

<210> 69
<211> 27
<212> DNA
<213> Artificial Sequence

<220>
<223> Synthetic oligonucleotide Probe

<400> 69
cctcctcctg ttgctgccac taatgct 27

<210> 70
<211> 20
<212> DNA
<213> Artificial Sequence

<220>
<223> Synthetic Oligonucleotide Forward Primer

<400> 70
aacttcaagg tcggagaagg 20

<210> 71
<211> 20
<212> DNA
<213> Artificial Sequence

<220>
<223> Synthetic Oligonucleotide Reverse Primer

<400> 71
tggctaaact cctgcacttg 20

<210> 72
<211> 21
<212> DNA
<213> Artificial Sequence

<220>
<223> Synthetic oligonucleotide Probe

<400> 72
ccgtccacgg tctcctcctc a 21

<210> 73

<211> 18
<212> DNA
<213> Artificial Sequence

<220>
<223> Synthetic oligonucleotide Foward Primer

<400> 73
gtgctacgcc accctgtt 18

<210> 74
<211> 20
<212> DNA
<213> Artificial Sequence

<220>
<223> synthetic oligonucleotide Reverse Primer

<400> 74
caggggcttc tcgtagatgt 20

<210> 75
<211> 22
<212> DNA
<213> Artificial Sequence

<220>
<223> synthetic oligonucleotide Probe

<400> 75
ccgatgttca cgcctttggg tc 22

<210> 76
<211> 19
<212> DNA
<213> Artificial Sequence

<220>
<223> Synthetic oligonucleotide Foward Primer

<400> 76
ggtgcctact ccattgtgg 19

<210> 77
<211> 20
<212> DNA
<213> Artificial sequence

<220>
<223> Synthetic Oligonucleotide Reverse Primer

<400> 77
gtggagccct tcttcctctt 20

<210> 78
<211> 24
<212> DNA
<213> Artificial Sequence

<220>
<223> Synthetic oligonucleotide Probe

<400> 78
tactccagca ggcacacaaa cacg 24

<210> 79
<211> 22
<212> DNA
<213> Artificial Sequence

<220>
<223> Synthetic oligonucleotide Forward Primer

<400> 79
gggagaacgg gatcaatagg at 22

<210> 80
<211> 21
<212> DNA
<213> Artificial Sequence

<220>
<223> Synthetic oligonucleotide Reverse Primer

<400> 80
gcatcagcca gtcctcaaac t 21

<210> 81
<211> 24
<212> DNA
<213> Artificial Sequence

<220>
<223> Synthetic oligonucleotide Probe

<400> 81
ctcattgggc accagcaggt ttcc 24

<210> 82
<211> 20
<212> DNA
<213> Artificial sequence

<220>
<223> synthetic oligonucleotide Forward Primer

<400> 82
tccaattcca gcatcactgt 20

<210> 83
<211> 20
<212> DNA
<213> Artificial Sequence

<220>
<223> Synthetic oligonucleotide Reverse Primer

<400> 83
ggcagtgaag gcgataaagt 20

<210> 84
<211> 25
<212> DNA
<213> Artificial Sequence

<220>
<223> synthetic oligonucleotide Probe

<400> 84
agaaaagctg tttgtctccc cagca 25

<210> 85
<211> 21
<212> DNA
<213> Artificial Sequence

<220>
<223> Synthetic oligonucleotide Forward Primer

<400> 85
tgcacagagg gtgtgggtta c 21

<210> 86
<211> 28
<212> DNA
<213> Artificial Sequence

<220>
<223> Synthetic Oligonucleotide Reverse Primer

<400> 86
tcttcatctg atttacaagc tgtacatg 28

<210> 87
<211> 29
<212> DNA
<213> Artificial Sequence

<220>
<223> Synthetic oligonucleotide Probe

<400> 87
caatgcttcc aacaatttgt ttgcttgcc 29

<210> 88
<211> 20
<212> DNA
<213> Artificial Sequence

<220>
<223> Synthetic oligonucleotide Forward Primer

<400> 88
actccctcta cccttgagca 20

<210> 89
<211> 20
<212> DNA
<213> Artificial sequence

<220>
<223> Synthetic oligonucleotide Reverse Primer

<400> 89
caggcctcag ttccttcagt 20

<210> 90
<211> 24
<212> DNA
<213> Artificial Sequence

<220>
<223> synthetic oligonucleotide Probe

<400> 90
cagaagaaca gctcagggac ccct 24

<210> 91
<211> 20
<212> DNA
<213> Artificial Sequence

<220>
<223> Synthetic oligonucleotide Forward Primer

<400> 91
tggtccatcg ccagttatca 20

<210> 92
<211> 23
<212> DNA
<213> Artificial sequence

<220>
<223> Synthetic oligonucleotide Reverse Primer

<400> 92
tgttctagcg atcttgcttc aca 23

<210> 93
<211> 30
<212> DNA
<213> Artificial Sequence

<220>
<223> Synthetic oligonucleotide Probe

<400> 93
atctgtatgc ggaacctcaa aagagtccct 30

<210> 94
<211> 23
<212> DNA

<213> Artificial Sequence

<220>
<223> Synthetic oligonucleotide Forward Primer

<400> 94
cggttatgtc atgccagata cac 23

<210> 95
<211> 24
<212> DNA
<213> Artificial Sequence

<220>
<223> Synthetic Oligonucleotide Reverse Primer

<400> 95
gaactgagac ccactgaaga aagg 24

<210> 96
<211> 25
<212> DNA
<213> Artificial sequence

<220>
<223> synthetic oligonucleotide Probe

<400> 96
cctcaaaggt actccctcct cccgg 25

<210> 97
<211> 25
<212> DNA
<213> Artificial Sequence

<220>
<223> synthetic oligonucleotide Forward Primer

<400> 97
tggctcttaa tcagtttcgt tacct 25

<210> 98
<211> 25
<212> DNA
<213> Artificial Sequence

<220>
<223> Synthetic oligonucleotide Reverse Primer

<400> 98
caaggcatat cgatcctcat aaagt 25

<210> 99
<211> 30
<212> DNA
<213> Artificial Sequence

<220>

<223> Synthetic Oligonucleotide Probe

<400> 99
tgtcccacga ataatgcgta aattctccag 30

<210> 100
<211> 20
<212> DNA
<213> Artificial sequence

<220>
<223> synthetic oligonucleotide Forward Primer

<400> 100
gtccaggtgg atgtgaaaga 20

<210> 101
<211> 20
<212> DNA
<213> Artificial Sequence

<220>
<223> synthetic oligonucleotide Reverse Primer

<400> 101
cggccaggat acacatctta 20

<210> 102
<211> 21
<212> DNA
<213> Artificial Sequence

<220>
<223> Synthetic oligonucleotide Probe

<400> 102
cagcaggccc tcaaggagct g 21

<210> 103
<211> 20
<212> DNA
<213> Artificial Sequence

<220>
<223> Synthetic Oligonucleotide Forward Primer

<400> 103
acggatcaca gtggaggaag 20

<210> 104
<211> 20
<212> DNA
<213> Artificial Sequence

<220>
<223> Synthetic oligonucleotide Reverse Primer

<400> 104

ctcatccgtc gggtcatagt 20

<210> 105
<211> 20
<212> DNA
<213> Artificial Sequence

<220>
<223> Synthetic Oligonucleotide Probe

<400> 105
cgctggctca cccctacctg 20

<210> 106
<211> 20
<212> DNA
<213> Artificial Sequence

<220>
<223> Synthetic oligonucleotide Forward Primer

<400> 106
ccagcaccat tgttgaagat 20

<210> 107
<211> 20
<212> DNA
<213> Artificial Sequence

<220>
<223> Synthetic oligonucleotide Reverse Primer

<400> 107
agtctcttgg gcatcgagtt 20

<210> 108
<211> 26
<212> DNA
<213> Artificial sequence

<220>
<223> Synthetic oligonucleotide Probe

<400> 108
ccccagacca agtgtgaata catgct 26

<210> 109
<211> 24
<212> DNA
<213> Artificial Sequence

<220>
<223> Synthetic oligonucleotide Forward Primer

<400> 109
gcactttggg attctttcca ttat 24

<210> 110

<211> 24
<212> DNA
<213> Artificial Sequence

<220>
<223> Synthetic oligonucleotide Reverse Primer

<400> 110
gcatgtaaga agaccctcac tgaa 24

<210> 111
<211> 29
<212> DNA
<213> Artificial Sequence

<220>
<223> Synthetic oligonucleotide Probe

<400> 111
acaacattct cggtgcctgt aacaaagaa 29

<210> 112
<211> 25
<212> DNA
<213> Artificial Sequence

<220>
<223> Synthetic oligonucleotide Reverse Primer

<400> 112
ggattgtaga ctgtcaccga aattc 25

<210> 113
<211> 28
<212> DNA
<213> Artificial Sequence

<220>
<223> Synthetic oligonucleotide Forward Primer

<400> 113
ggctattcct cattttctct acaaagtg 28

<210> 114
<211> 30
<212> DNA
<213> Artificial Sequence

<220>
<223> Synthetic oligonucleotide Probe

<400> 114
cctccaggag gctaccttct tcatgttcac 30

<210> 115
<211> 18
<212> DNA
<213> Artificial Sequence

<220>
<223> Synthetic oligonucleotide Forward Primer

<400> 115
ccagtggagc gcttccat 18

<210> 116
<211> 22
<212> DNA
<213> Artificial Sequence

<220>
<223> Synthetic Oligonucleotide Reverse Primer

<400> 116
ctctctgggt cgtctgaaac aa 22

<210> 117
<211> 23
<212> DNA
<213> Artificial Sequence

<220>
<223> Synthetic oligonucleotide Probe

<400> 117
tcggccactt catcaggacg cag 23

<210> 118
<211> 26
<212> DNA
<213> Artificial Sequence

<220>
<223> Synthetic oligonucleotide Forward Primer

<400> 118
ggataattca gacaacaaca ccatct 26

<210> 119
<211> 25
<212> DNA
<213> Artificial Sequence

<220>
<223> Synthetic oligonucleotide Reverse Primer

<400> 119
tgaagtaatc agccacagac tcaat 25

<210> 120
<211> 29
<212> DNA
<213> Artificial Sequence

<220>
<223> synthetic oligonucleotide Probe

<400> 120
tcaattgtaa cattctcacc caggccttg 29

<210> 121
<211> 20
<212> DNA
<213> Artificial Sequence

<220>
<223> Synthetic Oligonucleotide Forward Primer

<400> 121
gaagcgcaga tcatgaagaa 20

<210> 122
<211> 20
<212> DNA
<213> Artificial Sequence

<220>
<223> Synthetic Oligonucleotide Reverse Primer

<400> 122
ctcctcagac accactgcat 20

<210> 123
<211> 24
<212> DNA
<213> Artificial sequence

<220>
<223> Synthetic Oligonucleotide Probe

<400> 123
ctgaagcacg acaagctggt ccag 24

<210> 124
<211> 19
<212> DNA
<213> Artificial Sequence

<220>
<223> Synthetic oligonucleotide Forward Primer

<400> 124
tcagcagcaa gggcatcat 19

<210> 125
<211> 23
<212> DNA
<213> Artificial Sequence

<220>
<223> Synthetic oligonucleotide Reverse Primer

<400> 125
ggtggttttc ttgagcgtgt act 23

<210> 126
<211> 19
<212> DNA
<213> Artificial Sequence

<220>
<223> synthetic oligonucleotide Probe

<400> 126
cgcccgcagg cctcatcct 19

<210> 127
<211> 23
<212> DNA
<213> Artificial Sequence

<220>
<223> Synthetic oligonucleotide Forward Primer

<400> 127
caaaggagct cactgtggtg tct 23

<210> 128
<211> 26
<212> DNA
<213> Artificial Sequence

<220>
<223> Synthetic oligonucleotide Reverse Primer

<400> 128
gagtcagaat ggcttattca cagatg 26

<210> 129
<211> 24
<212> DNA
<213> Artificial Sequence

<220>
<223> Synthetic Oligonucleotide Probe

<400> 129
tgttccaacc actgaatctg gacc 24

<210> 130
<211> 20
<212> DNA
<213> Artificial Sequence

<220>
<223> Synthetic oligonucleotide Forward Primer

<400> 130
ttgggaaata tttgggcatt 20

<210> 131
<211> 20
<212> DNA

<213> Artificial Sequence

<220>
<223> Synthetic oligonucleotide Reverse Primer

<400> 131
agaagctagg gtggttgtcc 20

<210> 132
<211> 26
<212> DNA
<213> Artificial Sequence

<220>
<223> synthetic oligonucleotide Probe

<400> 132
ttgggacatt gtagacttgg ccagac 26

<210> 133
<211> 19
<212> DNA
<213> Artificial Sequence

<220>
<223> Synthetic Oligonucleotide Forward Primer

<400> 133
gcatgggaac catcaacca 19

<210> 134
<211> 21
<212> DNA
<213> Artificial Sequence

<220>
<223> Synthetic oligonucleotide Reverse Primer

<400> 134
tgaggagttt gccttgattc g 21

<210> 135
<211> 30
<212> DNA
<213> Artificial Sequence

<220>
<223> Synthetic Oligonucleotide Probe

<400> 135
ccatggacca acttcactat gtgacagagc 30

<210> 136
<211> 18
<212> DNA
<213> Artificial Sequence

<220>

<223> Synthetic oligonucleotide Forward Primer

<400> 136
ctccgacgtg gctttcca 18

<210> 137
<211> 23
<212> DNA
<213> Artificial Sequence

<220>
<223> Synthetic oligonucleotide Reverse Primer

<400> 137
cgtaattggc agaattgatg aca 23

<210> 138
<211> 25
<212> DNA
<213> Artificial Sequence

<220>
<223> Synthetic oligonucleotide Probe

<400> 138
tggcccacag catctatgga atccc 25

<210> 139
<211> 20
<212> DNA
<213> Artificial sequence

<220>
<223> Synthetic oligonucleotide Forward Primer

<400> 139
ccatctgcat ccatcttgtt 20

<210> 140
<211> 20
<212> DNA
<213> Artificial Sequence

<220>
<223> Synthetic oligonucleotide Reverse Primer

<400> 140
ggccaccagg gtattatctg 20

<210> 141
<211> 23
<212> DNA
<213> Artificial Sequence

<220>
<223> Synthetic oligonucleotide Probe

<400> 141

ctccccaccc ttgagaagtg cct 23

<210> 142
<211> 20
<212> DNA
<213> Artificial Sequence

<220>
<223> Synthetic oligonucleotide Forward Primer

<400> 142
aggctgctgg aggtcatctc 20

<210> 143
<211> 19
<212> DNA
<213> Artificial sequence

<220>
<223> Synthetic oligonucleotide Reverse Primer

<400> 143
cttcctgcgg ccacagtct 19

<210> 144
<211> 21
<212> DNA
<213> Artificial Sequence

<220>
<223> Synthetic oligonucleotide Probe

<400> 144
ccagaggccg tttcttggcc g 21

<210> 145
<211> 21
<212> DNA
<213> Artificial Sequence

<220>
<223> Synthetic oligonucleotide Forward Primer

<400> 145
tccatgatgg ttctgcaggt t 21

<210> 146
<211> 21
<212> DNA
<213> Artificial Sequence

<220>
<223> synthetic oligonucleotide Reverse Primer

<400> 146
tgagcagcac catcagtaac g 21

<210> 147

<211> 21
<212> DNA
<213> Artificial Sequence

<220>
<223> Synthetic Oligonucleotide Probe

<400> 147
ccccggacag tggctctgac g 21

<210> 148
<211> 23
<212> DNA
<213> Artificial Sequence

<220>
<223> Synthetic oligonucleotide Forward Primer

<400> 148
aacgactgct actccaagct caa 23

<210> 149
<211> 22
<212> DNA
<213> Artificial Sequence

<220>
<223> Synthetic Oligonucleotide Reverse Primer

<400> 149
ggatttccat cttgctcacc tt 22

<210> 150
<211> 22
<212> DNA
<213> Artificial Sequence

<220>
<223> synthetic oligonucleotide Probe

<400> 150
tgcccagcat cccccagaac aa 22

<210> 151
<211> 21
<212> DNA
<213> Artificial Sequence

<220>
<223> Synthetic Oligonucleotide Forward Primer

<400> 151
gcatggtagc cgaagatttc a 21

<210> 152
<211> 30
<212> DNA
<213> Artificial Sequence

<220>
<223> Synthetic oligonucleotide Reverse Primer

<400> 152
tttccggtaa tagtctgtct catagatatc 30

<210> 153
<211> 28
<212> DNA
<213> Artificial Sequence

<220>
<223> Synthetic oligonucleotide Probe

<400> 153
cgcgtcatac caaaatctcc gattttga 28

<210> 154
<211> 20
<212> DNA
<213> Artificial sequence

<220>
<223> Synthetic oligonucleotide Forward Primer

<400> 154
cctgaacctt ccaaagatgg 20

<210> 155
<211> 20
<212> DNA
<213> Artificial Sequence

<220>
<223> Synthetic oligonucleotide Reverse Primer

<400> 155
accaggcaag tctcctcatt 20

<210> 156
<211> 27
<212> DNA
<213> Artificial Sequence

<220>
<223> Synthetic oligonucleotide Probe

<400> 156
ccagattgga agcatccatc tttttca 27

<210> 157
<211> 20
<212> DNA
<213> Artificial sequence

<220>
<223> Synthetic oligonucleotide Forward Primer

<400> 157
agccatcact ctcagtgcag 20

<210> 158
<211> 20
<212> DNA
<213> Artificial sequence

<220>
<223> Synthetic oligonucleotide Reverse Primer

<400> 158
actgcagagt cagggtctcc 20

<210> 159
<211> 22
<212> DNA
<213> Artificial Sequence

<220>
<223> Synthetic oligonucleotide Probe

<400> 159
caggtcctat cgtggcccct ga 22

<210> 160
<211> 20
<212> DNA
<213> Artificial Sequence

<220>
<223> Synthetic Oligonucleotide Foward Primer

<400> 160
ccacagctca ccttctgtca 20

<210> 161
<211> 20
<212> DNA
<213> Artificial Sequence

<220>
<223> Synthetic Oligonucleotide Reverse Primer

<400> 161
cctcagtgcc agtctcttcc 20

<210> 162
<211> 20
<212> DNA
<213> Artificial Sequence

<220>
<223> synthetic Oligonucleotide Probe

<400> 162
tccatcccag ctccagccag 20

<210> 163
<211> 20
<212> DNA
<213> Artificial Sequence

<220>
<223> Synthetic Oligonucleotide Forward Primer

<400> 163
agagatcgag gctctcaagg 20

<210> 164
<211> 20
<212> DNA
<213> Artificial Sequence

<220>
<223> Synthetic Oligonucleotide Reverse Primer

<400> 164
ggccttttac ttcctcttcg 20

<210> 165
<211> 27
<212> DNA
<213> Artificial Sequence

<220>
<223> synthetic Oligonucleotide Probe

<400> 165
tggttcttct tcatgaagag cagctcc 27

<210> 166
<211> 23
<212> DNA
<213> Artificial sequence

<220>
<223> Synthetic oligonucleotide Forward Primer

<400> 166
tgagtggaaa agcctgacct atg 23

<210> 167
<211> 23
<212> DNA
<213> Artificial Sequence

<220>
<223> Synthetic Oligonucleotide Reverse Primer

<400> 167
ggactccatc tcttcttggt caa 23

<210> 168
<211> 27
<212> DNA

<213> Artificial Sequence

<220>
<223> Synthetic oligonucleotide Probe

<400> 168
tgaagtcatc agctttgtgc caccacc 27

<210> 169
<211> 21
<212> DNA
<213> Artificial Sequence

<220>
<223> synthetic Oligonucleotide Forward Primer

<400> 169
gacttttgcc cgctaccttt c 21

<210> 170
<211> 26
<212> DNA
<213> Artificial Sequence

<220>
<223> Synthetic oligonucleotide Reverse Primer

<400> 170
gccactaact gcttcagtat gaagag 26

<210> 171
<211> 24
<212> DNA
<213> Artificial Sequence

<220>
<223> synthetic Oligonucleotide Probe

<400> 171
acagctcatt gttgtcacgc cgga 24

<210> 172
<211> 24
<212> DNA
<213> Artificial Sequence

<220>
<223> Synthetic Oligonucleotide Forward Primer

<400> 172
tgatggtcct atgtgtcaca ttca 24

<210> 173
<211> 20
<212> DNA
<213> Artificial Sequence

<220>

<223> Synthetic Oligonucleotide Reverse Primer

<400> 173
tgggacagga aacacaccaa 20

<210> 174
<211> 30
<212> DNA
<213> Artificial Sequence

<220>
<223> synthetic oligonucleonde Probe

<400> 174
caggtttcat accaacacag gcttcagcac 30

<210> 175
<211> 19
<212> DNA
<213> Artificial Sequence

<220>
<223> Synthetic Oligonucleotide Forward Primer

<400> 175
cgctcagcca gatgcaatc 19

<210> 176
<211> 25
<212> DNA
<213> Artificial Sequence

<220>
<223> Synthetic Oligonucleotide Reverse Primer

<400> 176
gcactgagat cttcctattg gtgaa 25

<210> 177
<211> 21
<212> DNA
<213> Artificial sequence

<220>
<223> Synthetic Oligonucleotide Probe

<400> 177
tgccccagtc acctgctgtt a 21

<210> 178
<211> 20
<212> DNA
<213> Artificial Sequence

<220>
<223> Synthetic Oligonucleotide Forward Primer

<400> 178

gtgaaatgaa acgcaccaca 20

<210> 179
<211> 20
<212> DNA
<213> Artificial Sequence

<220>
<223> Synthetic oligonucleotide Reverse Primer

<400> 179
gaccctgctc acaaccagac 20

<210> 180
<211> 20
<212> DNA
<213> Artificial Sequence

<220>
<223> Synthetic Oligonucleotide Probe

<400> 180
cagcccttg gggaagctgg 20

<210> 181
<211> 19
<212> DNA
<213> Artificial Sequence

<220>
<223> synthetic Oligonucleotide Forward Primer

<400> 181
ccaacgcttg ccaaatcct 19

<210> 182
<211> 24
<212> DNA
<213> Artificial Sequence

<220>
<223> Synthetic Oligonucleotide Reverse Primer

<400> 182
acggtagtga cagcatcaaa actc 24

<210> 183
<211> 24
<212> DNA
<213> Artificial Sequence

<220>
<223> Synthetic Oligonucleotide Probe

<400> 183
aaccagctct ctgtgacccc aatt 24

<210> 184

<211> 21
<212> DNA
<213> Artificial Sequence

<220>
<223> Synthetic Oligonucleotide Forward Primer

<400> 184
tgattaccat catggctcag a 21

<210> 185
<211> 20
<212> DNA
<213> Artificial Sequence

<220>
<223> Synthetic oligonucleotide Reverse Primer

<400> 185
cttgtgaaaa tgccatccac 20

<210> 186
<211> 24
<212> DNA
<213> Artificial Sequence

<220>
<223> Synthetic Oligonucleotide Probe

<400> 186
tcccaattgt cgcttcttct gcag 24

<210> 187
<211> 19
<212> DNA
<213> Artificial Sequence

<220>
<223> Synthetic Oligonucleotide Forward Sequence

<400> 187
ccgccctcac ctgaagaga 19

<210> 188
<211> 22
<212> DNA
<213> Artificial sequence

<220>
<223> synthetic Oligonucleotide Reverse Sequence

<400> 188
ggaataagtt agccgcgctt ct 22

<210> 189
<211> 21
<212> DNA
<213> Artificial Sequence

<220>
<223> synthetic Oligonucleotide Probe

<400> 189
cccagtgtcc gccaaggagc g 21

<210> 190
<211> 20
<212> DNA
<213> Artificial Sequence

<220>
<223> Synthetic Oligonucleotide Forward Primer

<400> 190
ggccaggatc tgtggtggta 20

<210> 191
<211> 20
<212> DNA
<213> Artificial Sequence

<220>
<223> Synthetic Oligonucleotide Reverse Primer

<400> 191
ctccacgtcg tggacattga 20

<210> 192
<211> 23
<212> DNA
<213> Artificial sequence

<220>
<223> Synthetic Oligonucleotide Probe

<400> 192
ctctggcctt ccgagaaggt acc 23

<210> 193
<211> 20
<212> DNA
<213> Artificial Sequence

<220>
<223> Synthetic Oligonucleotide Forward Primer

<400> 193
ggctgtggct gaggctgtag 20

<210> 194
<211> 21
<212> DNA
<213> Artificial Sequence

<220>
<223> Synthetic Oligonucleotide Reverse Primer

<400> 194
ggagcattcg aggtcaaatc a 21

<210> 195
<211> 28
<212> DNA
<213> Artificial Sequence

<220>
<223> Synthetic Oligonucleotide Probe

<400> 195
ttcccagagt gtctcacctc cagcagag 28

<210> 196
<211> 20
<212> DNA
<213> Artificial Sequence

<220>
<223> Synthetic Oligonucleotide Forward Primer

<400> 196
gcatcaggct gtcattatgg 20

<210> 197
<211> 20
<212> DNA
<213> Artificial sequence

<220>
<223> Synthetic Oligonucleotide Reverse Primer

<400> 197
agtagttgtg ctgcccttcc 20

<210> 198
<211> 28
<212> DNA
<213> Artificial Sequence

<220>
<223> Synthetic Oligonucleotide Probe

<400> 198
tgtccttacc tgtgggagct gtaaggtc

<210> 199
<211> 20
<212> DNA
<213> Artificial Sequence

<220>
<223> Synthetic Oligonucleotide Forward Primer

<400> 199
ctgacacttg ccgcagagaa 20

<210> 200
<211> 20
<212> DNA
<213> Artificial Sequence

<220>
<223> Synthetic Oligonucleotide Reverse Primer

<400> 200
aggtggtcct tggtctggaa 20

<210> 201
<211> 26
<212> DNA
<213> Artificial Sequence

<220>
<223> Synthetic oligonucleotide Probe

<400> 201
ccctttctca cccacctcat ctgcac 26

<210> 202
<211> 23
<212> DNA
<213> Artificial sequence

<220>
<223> Synthetic Oligonucleotide Forward Primer

<400> 202
cgcttgccta actcatactt tcc 23

<210> 203
<211> 20
<212> DNA
<213> Artificial Sequence

<220>
<223> Synthetic Oligonucleotide Reverse Primer

<400> 203
ccattcagac tgcgccactt 20

<210> 204
<211> 19
<212> DNA
<213> Artificial Sequence

<220>
<223> Synthetic Oligonucleotide Probe

<400> 204
tccacgcagc gtggcactg 19

<210> 205
<211> 21
<212> DNA

<213> Artificial Sequence

<220>
<223> Synthetic Oligonucleotide Forward Primer

<400> 205
gcgacagctc ctctagttcc a 21

<210> 206
<211> 19
<212> DNA
<213> Artificial Sequence

<220>
<223> Synthetic Oligonucleotide Probe

<400> 206
ttcccgaagt ctccgcccg 19

<210> 207
<211> 22
<212> DNA
<213> Artificial Sequence

<220>
<223> Synthetic oligonucleotide Reverse Primer

<400> 207
ggaacaccag cttgaatttc ct 22

<210> 208
<211> 26
<212> DNA
<213> Artificial Sequence

<220>
<223> Synthetic Oligonucleotide Forward Primer

<400> 208
ggtgtcagat ataaatgtgc aaatgc 26

<210> 209
<211> 24
<212> DNA
<213> Artificial Sequence

<220>
<223> Synthetic Oligonucleotide Reverse Primer

<400> 209
ttcgatattg ccagcagcta taaa 24

<210> 210
<211> 28
<212> DNA
<213> Artificial Sequence

<220>

<223> Synthetic Oligonucleotide Probe

<400> 210
tgttgtcctg tcggtctcag tacgttca 28

<210> 211
<211> 20
<212> DNA
<213> Artificial Sequence

<220>
<223> Synthetic oligonucleotide Forward Primer

<400> 211
tgtggatgct ggattgattt 20

<210> 212
<211> 20
<212> DNA
<213> Artificial Sequence

<220>
<223> Synthetic oligonucleotide Reverse Primer

<400> 212
aagcagcact tcctggtctt 20

<210> 213
<211> 25
<212> DNA
<213> Artificial Sequence

<220>
<223> Synthetic Oligonucleotide Probe

<400> 213
ccactggtgc agctgctaaa tagca 25

<210> 214
<211> 20
<212> DNA
<213> Artificial Sequence

<220>
<223> Synthetic Oligonucleotide Forward Primer

<400> 214
agtgggagac acctgacctt 20

<210> 215
<211> 20
<212> DNA
<213> Artificial Sequence

<220>
<223> Synthetic Oligonucleotide Reverse Primer

<400> 215

tgatctgggc attgtactcc 20

<210> 216
<211> 29
<212> DNA
<213> Artificial Sequence

<220>
<223> Synthetic Oligonucleotide Probe

<400> 216
ttgatcttct gctcaatctc agcttgaga 29

<210> 217
<211> 18
<212> DNA
<213> Artificial Sequence

<220>
<223> synthetic Oligonucleotide Forward Primer

<400> 217
cccgttcggt ctgaggaa 18

<210> 218
<211> 22
<212> DNA
<213> Artificial Sequence

<220>
<223> Synthetic Oligonucleotide Reverse Primer

<400> 218
gagcactcaa ggtagccaaa gg 22

<210> 219
<211> 19
<212> DNA
<213> Artificial sequence

<220>
<223> synthetic Oligonucleotide Probe

<400> 219
tccggttcgc catgtcccg 19

<210> 220
<211> 20
<212> DNA
<213> Artificial Sequence

<220>
<223> Synthetic Oligonucleotide Forward Primer

<400> 220
aacagagaca ttgccaacca 20

<210> 221

<211> 20
<212> DNA
<213> Artificial Sequence

<220>
<223> Synthetic oligonucleotide Reverse Primer

<400> 221
gtgatttgcc caggaagttt 20

<210> 222
<211> 24
<212> DNA
<213> Artificial Sequence

<220>
<223> Synthetic oligonucleotide Probe

<400> 222
ttggatctgc ttgctgtcca aacc 24

<210> 223
<211> 20
<212> DNA
<213> Artificial sequence

<220>
<223> Synthetic Oligonucleotide Forward Primer

<400> 223
cgcgagcccc tcattataca 20

<210> 224
<211> 19
<212> DNA
<213> Artificial sequence

<220>
<223> synthetic Oligonucleotide Reverse Primer

<400> 224
cactcgccgt tgacatcct 19

<210> 225
<211> 22
<212> DNA
<213> Artificial sequence

<220>
<223> synthetic oligonucleotide Probe

<400> 225
ctccccacag cgcatcgagg aa 22

<210> 226
<211> 23
<212> DNA
<213> Artificial Sequence

<220>
<223> Synthetic Oligonucleotide Forward Primer

<400> 226
cagtggagac cagttgggta gtg 23

<210> 227
<211> 20
<212> DNA
<213> Artificial Sequence

<220>
<223> Synthetic Oligonucleotide Reverse Primer

<400> 227
ccttgaagag cgtcccatca 20

<210> 228
<211> 28
<212> DNA
<213> Artificial Sequence

<220>
<223> Synthetic oligonucleotide Probe

<400> 228
cacacatgca gagcttgtag cgtaccca 28

<210> 229
<211> 20
<212> DNA
<213> Artificial sequence

<220>
<223> Synthetic Oligonucleotide Forward Primer

<400> 229
gggctcagct ttcagaagtg 20

<210> 230
<211> 20
<212> DNA
<213> Artificial Sequence

<220>
<223> synthetic Oligonucleotide Reverse Primer

<400> 230
acatgttcag ctggtccaca 20

<210> 231
<211> 25
<212> DNA
<213> Artificial Sequence

<220>
<223> synthetic oligonucleotide Probe

<400> 231
tggcagtttt cttctgtcac caaaa 25

<210> 232
<211> 20
<212> DNA
<213> Artificial Sequence

<220>
<223> Synthetic Oligonucleotide Forward Primer

<400> 232
tcacatgcca ctttggtgtt 20

<210> 233
<211> 20
<212> DNA
<213> Artificial Sequence

<220>
<223> Synthetic Oligonucleotide Reverse Primer

<400> 233
cttgcaggaa gcggctatac 20

<210> 234
<211> 22
<212> DNA
<213> Artificial Sequence

<220>
<223> Synthetic oligonucleotide Probe

<400> 234
tcctgggaga gattgaccag ca 22

<210> 235
<211> 19
<212> DNA
<213> Artificial Sequence

<220>
<223> Synthetic Oligonucleotide Forward Primer

<400> 235
gcccgaaacg ccgaatata 19

<210> 236
<211> 23
<212> DNA
<213> Artificial Sequence

<220>
<223> Synthetic Oligonucleotide Reverse Primer

<400> 236
cgtggctctc ttatcctcat gat 23

<210> 237
<211> 21
<212> DNA
<213> Artificial Sequence

<220>
<223> Synthetic Oligonucleotide Probe

<400> 237
taccgcagca aaccgcttgg g 21

<210> 238
<211> 20
<212> DNA
<213> Artificial Sequence

<220>
<223> Synthetic Oligonucleotide Forward Primer

<400> 238
gccgggaaga ccgtaattgt 20

<210> 239
<211> 18
<212> DNA
<213> Artificial Sequence

<220>
<223> Synthetic Oligonucleotide Reverse Primer

<400> 239
cagcggcacc aggttcag 18

<210> 240
<211> 26
<212> DNA
<213> Artificial Sequence

<220>
<223> Synthetic Oligonucleotide Probe

<400> 240
caaatggctt cctctggaag gtccca 26

<210> 241
<211> 20
<212> DNA
<213> Artificial sequence

<220>
<223> synthetic Oligonucleotide Forward Primer

<400> 241
tgctgttgct gagtctgttg 20

<210> 242
<211> 20
<212> DNA

<213> Artificial sequence

<220>
<223> Synthetic oligonucleotide Reverse Primer

<400> 242
cttgcctggc ttcacagata 20

<210> 243
<211> 24
<212> DNA
<213> Artificial Sequence

<220>
<223> Synthetic Oligonucleotide Probe

<400> 243
ccagtcccca gaagaccatg tctg 24

<210> 244
<211> 20
<212> DNA
<213> Artificial Sequence

<220>
<223> Synthetic oligonucleotide Forward Primer

<400> 244
tgtggtgagg aaggagtcag 20

<210> 245
<211> 18
<212> DNA
<213> Artificial sequence

<220>
<223> Synthetic Oligonucleotide Reverse Primer

<400> 245
cccagagagt gggtcagc 18

<210> 246
<211> 24
<212> DNA
<213> Artificial Sequence

<220>
<223> Synthetic Oligonucleotide Probe

<400> 246
ctgtgactgt ctccagggct tcca 24

<210> 247
<211> 21
<212> DNA
<213> Artificial sequence

<220>

<223> Synthetic Oligonucleotide Forward Primer

<400> 247
tggcttcagg agctgaatac c 21

<210> 248
<211> 24
<212> DNA
<213> Artificial Sequence

<220>
<223> Synthetic Oligonucleotide Reverse Primer

<400> 248
tgctgtcgtg atgagaaaat agtg 24

<210> 249
<211> 26
<212> DNA
<213> Artificial sequence

<220>
<223> Synthetic Oligonucleotide Probe

<400> 249
caggcacaca caggtgggac acaaat 26

<210> 250
<211> 25
<212> DNA
<213> Artificial Sequence

<220>
<223> Synthetic Oligonucleotide Forward Primer

<400> 250
gtatcaggac cacatgcagt acatc 25

<210> 251
<211> 22
<212> DNA
<213> Artificial Sequence

<220>
<223> Synthetic Oligonucleotide Reverse Primer

<400> 251
tgtcggaatt gatactggca tt 22

<210> 252
<211> 24
<212> DNA
<213> Artificial sequence

<220>
<223> Synthetic oligonucleotide Probe

<400> 252

ttgatgcctg tcttcgcgcc ttct 24

<210> 253
<211> 20
<212> DNA
<213> Artificial Sequence

<220>
<223> Synthetic Oligonucleotide Forward Primer

<400> 253
tttccaaaca tcagcgagtc 20

<210> 254
<211> 20
<212> DNA
<213> Artificial Sequence

<220>
<223> synthetic Oligonucleotide Reverse Primer

<400> 254
aacaggagcg cttgaaagtt 20

<210> 255
<211> 23
<212> DNA
<213> Artificial Sequence

<220>
<223> Synthetic Oligonucleotide Probe

<400> 255
acggtgcttc tccctctcca gtg 23

<210> 256
<211> 78
<212> DNA
<213> Artificial Sequence

<220>
<223> Amplicon

<400> 256

cgttccgatc ctctatactg catcccaggc atgcctacag caccctgatg tcgcagccta 60
taaggccaac agggacct 78

<210> 257
<211> 76
<212> DNA
<213> Artificial Sequence

<220>
<223> Amplicon

<400> 257

tgcagactgt accatgctga ccattgccca tcgcctgcac acggttctag gctccgatag 60
gattatggtg ctggcc 76

<210> 258
<211> 78
<212> DNA
<213> Artificial Sequence

<220>
<223> Amplicon

<400> 258

ctgcatgtga ttgaataaga aacaagaaag tgaccacacc aaagcctccc tggctggtgt 60
acagggatca ggtccaca 78

<210> 259
<211> 68
<212> DNA
<213> Artificial Sequence

<220>
<223> Amplicon

<400> 259

acgaattgtc ggtgaggtct caggatacca cagtcctgga gaccctatcc aatgatttca 60
gcatggac 68

<210> 260
<211> 71
<212> DNA
<213> Artificial Sequence

<220>
<223> Amplicon

<400> 260

cgcttctatg gcgctgagat tgtgtcagcc ctggactacc tgcactcgga gaagaacgtg 60
gtgtaccggg a 71

<210> 261
<211> 71
<212> DNA
<213> Artificial Sequence

<220>
<223> Amplicon

<400> 261

```
tcctgccacc cttcaaacct caggtcacgt ccgaggtcga cacaaggtac ttcgatgatg 60
aatttaccgc c                                                      71
```

<210> 262
<211> 69
<212> DNA
<213> Artificial Sequence

<220>
<223> Amplicon

<400> 262

```
ggacagcagg aatgtgtttc tccatacagg tcacggggag ccaatggttc agaaacaaat 60
cgagtgggt                                                         69
```

<210> 263
<211> 73
<212> DNA
<213> Artificial Sequence

<220>
<223> Amplicon

<400> 263

```
gggtcaggtg cctcgagatc gggcttgggc ccagagcatg ttccagatcc cagagtttga 60
gccgagtgag cag                                                    73
```

<210> 264
<211> 81
<212> DNA
<213> Artificial sequence

<220>
<223> Amplicon

<400> 264

```
cgttgtcagc acttggaata caagatggtt gccgggtcat gttaattggg aaaaagaaca 60
gtccacagga agaggttgaa c                                           81
```

<210> 265
<211> 83
<212> DNA
<213> Artificial Sequence

<220>
<223> Amplicon

<400> 265

cctggagggt cctgtacaat ctcatcatgg gactcctgcc cttacccagg ggccacagag 60
cccccgagat ggagcccaat tag 83

<210> 266
<211> 73
<212> DNA
<213> Artificial Sequence

<220>
<223> Amplicon

<400> 266

cagatggacc tagtacccac tgagatttcc acgccgaagg acagcgatgg gaaaaatgcc 60
cttaaatcat agg 73

<210> 267
<211> 69
<212> DNA
<213> Artificial sequence

<220>
<223> Amplicon

<400> 267

gcatgttcgt ggcctctaag atgaaggaga ccatccccct gacggccgag aagctgtgca 60
tctacaccg 69

<210> 268
<211> 81
<212> DNA
<213> Artificial Sequence

<220>
<223> Amplicon

<400> 268

cgtcaggacc caccatgtct gccccatcac gcggccgaga catggcttgg ccacagctct 60

tgaggatgtc accaattaac c 81

<210> 269
<211> 75
<212> DNA
<213> Artificial Sequence

<220>
<223> Amplicon

<400> 269

tgtatttcaa gacctctgtg cacttattta tgaacctgcc ctgctcccac agaacacagc 60
aattcctcag gctaa 75

<210> 270
<211> 65
<212> DNA
<213> Artificial Sequence

<220>
<223> Amplicon

<400> 270

agatgaagtg gaaggcgctt ttcaccgcgg ccatcctgca ggcacagttg ccgattacag 60
aggca 65

<210> 271
<211> 64
<212> DNA
<213> Artificial Sequence

<220>
<223> Amplicon

<400> 271

gggcgtggaa cagtttatct cagacatctg ccccaagaag gacgtactcg aaaccttcac 60
cgtg 64

<210> 272
<211> 68
<212> DNA
<213> Artificial Sequence

<220>
<223> Amplicon

<400> 272

tggattggag ttctgggaat gtactggccg tggcactgga caacagtgtg tacctgtgga 60
gtgcaagc 68

<210> 273
<211> 85
<212> DNA
<213> Artificial Sequence

<220>
<223> Amplicon

<400> 273

aaacgagcag tttgccatca gacgcttcca gtctatgccg gtgaggctgc tgggccacag 60
ccccgtgctt cggaacatca ccaac 85

<210> 274
<211> 77
<212> DNA
<213> Artificial Sequence

<220>
<223> Amplicon

<400> 274

tgacaatcag cacacctgca ttcaccgctc ggaagagggc ctgagctgca tgaataagga 60
tcacggctgt agtcaca 77

<210> 275
<211> 76
<212> DNA
<213> Artificial Sequence

<220>
<223> Amplicon

<400> 275

ctgaaggagc tccaagacct cgctctccaa ggcgccaagg agagggcaca tcagcagaag 60
aaacacagcg gttttg 76

<210> 276
<211> 68
<212> DNA
<213> Artificial Sequence

<220>
<223> Amplicon

<400> 276

gtggccatcc agctgacctt cctgcgcctg atgtccaccg aggcctccca gaacatcacc 60
taccactg 68

<210> 277
<211> 80
<212> DNA
<213> Artificial Sequence

<220>
<223> Amplicon

<400> 277

```
cagccaagaa ctggtatagg agctccaagg acaagaaaca cgtctggcta ggagaaacta 60
tcaatgctgg cagccagttt                                             80
```

<210> 278
<211> 81
<212> DNA
<213> Artificial Sequence

<220>
<223> Amplicon

<400> 278

```
cgacagttgc gatgaaagtt ctaatctctt ccctcctcct gttgctgcca ctaatgctga 60
tgtccatggt ctctagcagc c                                           81
```

<210> 279
<211> 67
<212> DNA
<213> Artificial Sequence

<220>
<223> Amplicon

<400> 279

```
aacttcaagg tcggagaagg ctttgaggag gagaccgtgg acggacgcaa gtgcaggagt 60
ttagcca                                                           67
```

<210> 280
<211> 76
<212> DNA
<213> Artificial Sequence

<220>
<223> Amplicon

<400> 280

```
gtgctacgcc accctgttcg gacccaaagg cgtgaacatc gggggcgcgg gctcctacat 60
ctacgagaag cccctg                                                 76
```

<210> 281
<211> 77
<212> DNA
<213> Artificial Sequence

<220>
<223> Amplicon

<400> 281

ggtgcctact ccattgtggc gggcgtgttt gtgtgcctgc tggagtaccc ccgggggaag 60
aggaagaagg gctccac 77

<210> 282
<211> 78
<212> DNA
<213> Artificial Sequence

<220>
<223> Amplicon

<400> 282

gggagaacgg gatcaatagg atcggaaacc tgctggtgcc caatgagaat tactgcaagt 60
ttgaggactg gctgatgc 78

<210> 283
<211> 68
<212> DNA
<213> Artificial Sequence

<220>
<223> Amplicon

<400> 283

tccaattcca gcatcactgt ggagaaaagc tgtttgtctc cccagcatac tttatcgcct 60
tcactgcc 68

<210> 284
<211> 83
<212> DNA
<213> Artificial Sequence

<220>
<223> Amplicon

<400> 284

tgcacagagg gtgtgggtta caccaatgct tccaacaatt tgtttgcttg cctcccatgt 60
acagcttgta aatcagatga aga 83

<210> 285
<211> 75
<212> DNA
<213> Artificial sequence

<220>
<223> Amplicon

<400> 285

actccctcta cccttgagca agggcagggg tccctgagct gttcttctgc cccatactga 60

aggaactgag gcctg 75

<210> 286
<211> 76
<212> DNA
<213> Artificial Sequence

<220>
<223> Amplicon

<400> 286

tggtccatcg ccagttatca catctgtatg cggaacctca aaagagtccc tggtgtgaag 60
caagatcgct agaaca 76

<210> 287
<211> 81
<212> DNA
<213> Artificial Sequence

<220>
<223> Amplicon

<400> 287

cggttatgtc atgccagata cacacctcaa aggtactccc tcctcccggg aaggcaccct 60
ttcttcagtg ggtctcagtt c 81

<210> 288
<211> 86
<212> DNA
<213> Artificial Sequence

<220>
<223> Amplicon

<400> 288

tggctcttaa tcagtttcgt tacctgcctc tggagaattt acgcattatt cgtgggacaa 60
aactttatga ggatcgatat gccttg 86

<210> 289
<211> 67
<212> DNA
<213> Artificial Sequence

<220>
<223> Amplicon

<400> 289

gtccaggtgg atgtgaaaga tccccagcag gccctcaagg agctggctaa gatgtgtatc 60
ctggccg 67

<210> 290
<211> 67
<212> DNA
<213> Artificial Sequence

<220>
<223> Amplicon

<400> 290

```
acggatcaca gtggaggaag cgctggctca cccctacctg gagcagtact atgacccgac 60
ggatgag                                                           67
```

<210> 291
<211> 67
<212> DNA
<213> Artificial Sequence

<220>
<223> Amplicon

<400> 291

```
ccagcaccat tgttgaagat ccccagacca agtgtgaata catgctcaac tcgatgccca 60
agagact                                                           67
```

<210> 292
<211> 80
<212> DNA
<213> Artificial sequence

<220>
<223> Amplicon

<400> 292

```
gcactttggg attctttcca ttatgattct ttgttacagg caccgagaat gttgtattca 60
gtgagggtct tcttacatgc                                             80
```

<210> 293
<211> 90
<212> DNA
<213> Artificial Sequence

<220>
<223> Amplicon

<400> 293

```
ggctattcct cattttctct acaaagtggc ctcagtgaac atgaagaagg tagcctcctg 60
gaggagaatt tcggtgacag tctacaatcc                                  90
```

<210> 294
<211> 67

<212> DNA
<213> Artificial Sequence

<220>
<223> Amplicon

<400> 294

ccagtggagc gcttccatga cctgcgtcct gatgaagtgg ccgatttgtt tcagacgacc 60
cagagag 67

<210> 295
<211> 80
<212> DNA
<213> Artificial Sequence

<220>
<223> Amplicon

<400> 295

ggataattca gacaacaaca ccatctttgt gcaaggcctg ggtgagaatg ttacaattga 60
gtctgtggct gattacttca 80

<210> 296
<211> 69
<212> DNA
<213> Artificial Sequence

<220>
<223> Amplicon

<400> 296

gaagcgcaga tcatgaagaa gctgaagcac gacaagctgg tccagctcta tgcagtggtg 60
tctgaggag 69

<210> 297
<211> 68
<212> DNA
<213> Artificial Sequence

<220>
<223> Amplicon

<400> 297

tcagcagcaa gggcatcatg gaggaggatg aggcctgcgg gcgccagtac acgctcaaga 60
aaaccacc 68

<210> 298
<211> 75
<212> DNA
<213> Artificial Sequence

<220>
<223> Amplicon

<400> 298

```
caaaggagct cactgtggtg tctgtgttcc aaccactgaa tctggacccc atctgtgaat 60
aagccattct gactc                                                  75
```

<210> 299
<211> 73
<212> DNA
<213> Artificial Sequence

<220>
<223> Amplicon

<400> 299

```
ttgggaaata tttgggcatt ggtctggcca agtctacaat gtcccaatat caaggacaac 60
caccctagct tct                                                    73
```

<210> 300
<211> 83
<212> DNA
<213> Artificial Sequence

<220>
<223> Amplicon

<400> 300

```
gcatgggaac catcaaccag caggccatgg accaacttca ctatgtgaca gagctgacag 60
atcgaatcaa ggcaaactcc tca                                         83
```

<210> 301
<211> 70
<212> DNA
<213> Artificial sequence

<220>
<223> Amplicon

<400> 301

```
ctccgacgtg gctttccagt ggcccacagc atctatggaa tcccatctgt catcaattct 60
gccaattacg                                                        70
```

<210> 302
<211> 67
<212> DNA
<213> Artificial Sequence

<220>
<223> Amplicon

<400> 302

ccatctgcat ccatcttgtt tgggctcccc acccttgaga agtgcctcag ataataccct 60

ggtggcc 67

<210> 303
<211> 84
<212> DNA
<213> Artificial Sequence

<220>
<223> Amplicon

<400> 303

aggctgctgg aggtcatctc cgtgtgtgat tgccccagag gccgtttctt ggccgccatc 60
tgccaagact gtggccgcag gaag 84

<210> 304
<211> 73
<212> DNA
<213> Artificial Sequence

<220>
<223> Amplicon

<400> 304

tccatgatgg ttctgcaggt ttctgcggcc ccccggacag tggctctgac ggcgttactg 60
atggtgctgc tca 73

<210> 305
<211> 76
<212> DNA
<213> Artificial Sequence

<220>
<223> Amplicon

<400> 305

aacgactgct actccaagct caaggagctg gtgcccagca tcccccagaa caagaaggtg 60
agcaagatgg aaatcc 76

<210> 306
<211> 83
<212> DNA
<213> Artificial Sequence

<220>
<223> Amplicon

<400> 306

```
gcatggtagc cgaagatttc acagtcaaaa tcggagattt tggtatgacg cgagatatct 60
atgagacaga ctattaccgg aaa                                         83
```

<210> 307
<211> 72
<212> DNA
<213> Artificial Sequence

<220>
<223> Amplicon

<400> 307

```
cctgaacctt ccaaagatgg ctgaaaaaga tggatgcttc caatctggat tcaatgagga 60
gacttgcctg gt                                                     72
```

<210> 308
<211> 63
<212> DNA
<213> Artificial Sequence

<220>
<223> Amplicon

<400> 308

```
agccatcact ctcagtgcag ccaggtccta tcgtggcccc tgaggagacc ctgactctgc 60
agt                                                               63
```

<210> 309
<211> 74
<212> DNA
<213> Artificial Sequence

<220>
<223> Amplicon

<400> 309

```
ccacagctca ccttctgtca ggtgtccatc ccagctccag ccagctccca gagaggaaga 60
gactggcact gagg                                                   74
```

<210> 310
<211> 68
<212> DNA
<213> Artificial Sequence

<220>
<223> Amplicon

<400> 310

agagatcgag gctctcaagg aggagctgct cttcatgaag aagaaccacg aagaggaagt 60
aaaaggcc 68

<210> 311
<211> 76
<212> DNA
<213> Artificial Sequence

<220>
<223> Amplicon

<400> 311

tgagtggaaa agcctgacct atgatgaagt catcagcttt gtgccaccac cccttgacca 60
agaagagatg gagtcc 76

<210> 312
<211> 75
<212> DNA
<213> Artificial Sequence

<220>
<223> Amplicon

<400> 312

gacttttgcc cgctaccttt cattccggcg tgacaacaat gagctgttgc tcttcatact 60
gaagcagtta gtggc 75

<210> 313
<211> 82
<212> DNA
<213> Artificial Sequence

<220>
<223> Amplicon

<400> 313

tgatggtcct atgtgtcaca ttcatcacag gtttcatacc aacacaggct tcagcacttc 60
ctttggtgtg tttcctgtcc ca 82

<210> 314
<211> 71
<212> DNA
<213> Artificial Sequence

<220>
<223> Amplicon

<400> 314

cgctcagcca gatgcaatca atgccccagt cacctgctgt tataacttca ccaataggaa 60
gatctcagtg c 71

<210> 315
<211> 69
<212> DNA
<213> Artificial Sequence

<220>
<223> Amplicon

<400> 315

gtgaaatgaa acgcaccaca ctggacagcc ctttggggaa gctggagctg tctggttgtg 60
agcagggtc 69

<210> 316
<211> 78
<212> DNA
<213> Artificial Sequence

<220>
<223> Amplicon

<400> 316

ccaacgcttg ccaaatcctg acaattcaga accagctctc tgtgacccca atttgagttt 60
tgatgctgtc actaccgt 78

<210> 317
<211> 82
<212> DNA
<213> Artificial Sequence

<220>
<223> Amplicon

<400> 317

tgattaccat catggctcag attggctcct atgttcctgc agaagaagcg acaattggga 60
ttgtggatgg cattttcaca ag 82

<210> 318
<211> 77
<212> DNA
<213> Artificial Sequence

<220>
<223> Amplicon

<400> 318

ccgccctcac ctgaagagaa acgcgctcct tggcggacac tgggggagga gaggaagaag 60
cgcggctaac ttattcc 77

<210> 319
<211> 71
<212> DNA
<213> Artificial Sequence

<220>
<223> Amplicon

<400> 319

ggccaggatc tgtggtggta caattgactc tggccttccg agaaggtacc atcaatgtcc 60

acgacgtgga g 71

<210> 320
<211> 73
<212> DNA
<213> Artificial Sequence

<220>
<223> Amplicon

<400> 320

ggctgtggct gaggctgtag catctctgct ggaggtgaga cactctggga actgatttga 60
cctcgaatgc tcc 73

<210> 321
<211> 85
<212> DNA
<213> Artificial Sequence

<220>
<223> Amplicon

<400> 321

gcatcaggct gtcattatgg tgtccttacc tgtgggagct gtaaggtctt ctttaagagg 60
gcaatggaag ggcagcacaa ctact 85

<210> 322
<211> 68
<212> DNA
<213> Artificial Sequence

<220>
<223> Amplicon

<400> 322

ctgacacttg ccgcagagaa tccctttctc acccacctca tctgcacctt ccagaccaag 60
gaccacct 68

<210> 323
<211> 81
<212> DNA
<213> Artificial sequence

<220>
<223> Amplicon

<400> 323

cgcttgccta actcatactt tcccgttgac acttgatcca cgcagcgtgg cactgggacg 60
taagtggcgc agtctgaatg g 81

<210> 324
<211> 78
<212> DNA
<213> Artificial sequence

<220>
<223> Amplicon

<400> 324

gcgacagctc ctctagttcc accatgtccg cgggcggaga cttcgggaat ccgctgagga 60
aattcaagct ggtgttcc 78

<210> 325
<211> 84
<212> DNA
<213> Artificial Sequence

<220>
<223> Amplicon

<400> 325

ggtgtcagat ataaatgtgc aaatgccttc ttgctgtcct gtcggtctca gtacgttcac 60
tttatagctg ctggcaatat cgaa 84

<210> 326
<211> 67
<212> DNA
<213> Artificial Sequence

<220>
<223> Amplicon

<400> 326

tgtggatgct ggattgattt caccactggt gcagctgcta aatagcaaag accaggaagt 60
gctgctt 67

<210> 327
<211> 69
<212> DNA
<213> Artificial Sequence

<220>
<223> Amplicon

<400> 327

agtgggagac acctgacctt tctcaagctg agattgagca gaagatcaag gagtacaatg 60
cccagatca 69

<210> 328
<211> 68
<212> DNA
<213> Artificial Sequence

<220>
<223> Amplicon

<400> 328

cccgttcggt ctgaggaagg ccgggacatg gcgaaccgga tcagtgcctt tggctacctt 60
gagtgctc 68

<210> 329
<211> 69
<212> DNA
<213> Artificial sequence

<220>
<223> Amplicon

<400> 329

aacagagaca ttgccaacca tattggatct gcttgctgtc caaaccagca aacttcctgg 60
gcaaatcac 69

<210> 330
<211> 86
<212> DNA
<213> Artificial Sequence

<220>
<223> Amplicon

<400> 330

cgcgagcccc tcattataca ctgggcagcc tccccacagc gcatcgagga atgcgtgctc 60
tcaggcaagg atgtcaacgg cgagtg 86

<210> 331
<211> 77
<212> DNA
<213> Artificial Sequence

<220>
<223> Amplicon

<400> 331

cagtggagac cagttgggta gtggtgactg ggtacgctac aagctctgca tgtgtgctga 60
tgggacgctc ttcaagg 77

<210> 332
<211> 81
<212> DNA
<213> Artificial Sequence

<220>
<223> Amplicon

<400> 332

gggctcagct ttcagaagtg ctgagttggc agttttcttc tgtcaccaaa agaggtctca 60
atgtggacca gctgaacatg t 81

<210> 333
<211> 70
<212> DNA
<213> Artificial Sequence

<220>
<223> Amplicon

<400> 333

tcacatgcca ctttggtgtt tcataatctc ctgggagaga ttgaccagca gtatagccgc 60
ttcctgcaag 70

<210> 334
<211> 65
<212> DNA
<213> Artificial Sequence

<220>
<223> Amplicon

<400> 334

gcccgaaacg ccgaatataa tcccaagcgg tttgctgcgg taatcatgag gataagagag 60
ccacg 65

<210> 335
<211> 84
<212> DNA
<213> Artificial sequence

<220>
<223> Amplicon

<400> 335

gccgggaaga ccgtaattgt ggctgcactg gatgggacct tccagaggaa gccatttggg 60
gccatcctga acctggtgcc gctg 84

<210> 336
<211> 74
<212> DNA
<213> Artificial Sequence

<220>
<223> Amplicon

<400> 336

tgctgttgct gagtctgttg ccagtcccca gaagaccatg tctgtgttga gctgtatctg 60

tgaagccagg caag 74

<210> 337
<211> 66
<212> DNA
<213> Artificial Sequence

<220>
<223> Amplicon

<400> 337

tgtggtgagg aaggagtcag agagctgtga ctgtctccag ggcttccagc tgacccactc 60
tctggg 66

<210> 338
<211> 89
<212> DNA
<213> Artificial Sequence

<220>
<223> Amplicon

<400> 338

tggcttcagg agctgaatac cctcccaggc acacacaggt gggacacaaa taagggtttt 60
ggaaccacta ttttctcatc acgacagca 89

<210> 339
<211> 75
<212> DNA
<213> Artificial Sequence

<220>
<223> Amplicon

<400> 339

gtatcaggac cacatgcagt acatcggaga aggcgcgaag acaggcatca aagaatgcca 60
gtatcaattc cgaca 75

<210> 340
<211> 65
<212> DNA
<213> Artificial Sequence

<220>
<223> Amplicon

<400> 340

tttccaaaca tcagcgagtc cacactggag agggagaagc accgtaactt tcaagcgctc 60
ctgtt 65

## Description

### Background of the Invention

### Field of the Invention

The present invention provides sets of genes the expression of which is important in the prognosis of cancer. In particular, the invention provides gene expression information useful for predicting whether cancer patients are likely to have a beneficial treatment response to chemotherapy.

### Description of the Related Art

Oncologists have a number of treatment options available to them, including different combinations of chemotherapeutic drugs that are characterized as "standard of care," and a number of drugs that do not carry a label claim for particular cancer, but for which there is evidence of efficacy in that cancer. Best likelihood of good treatment outcome requires that patients be assigned to optimal available cancer treatment, and that this assignment be made as quickly as possible following diagnosis. In particular, it is important to determine the likelihood of patient response to "standard of care" chemotherapy because chemotherapeutic drugs such as anthracyclines and taxanes have limited efficacy and are toxic. The identification of patients who are most or least likely to respond thus could increase the net benefit these drugs have to offer, and decrease the net morbidity and toxicity, via more intelligent patient selection.

Currently, diagnostic tests used in clinical practice are single analyte, and therefore do not capture the potential value of knowing relationships between dozens of different markers. Moreover, diagnostic tests are frequently not quantitative, relying on immunohistochemistry. This method often yields different results in different laboratories, in part because the reagents are not standardized, and in part because the interpretations are subjective and cannot be easily quantified. RNA-based tests have not often been used because of the problem of RNA degradation over time and the fact that it is difficult to obtain fresh tissue samples from patients for analysis. Fixed paraffin-embedded tissue is more readily available and methods have been established to detect RNA in fixed tissue. However, these methods typically do not allow for the study of large numbers of genes (DNA or RNA) from small amounts of material. Thus, traditionally fixed tissue has been rarely used other than for immunohistochhemistry detection of proteins.

In the last few years, several groups have published studies concerning the classification of various cancer types by microarray gene expression analysis (see, e.g. Golub et al., Science 286:531-537 (1999); Bhattacharjae et al., Proc. Natl. Acad. Sci. USA 98:13790-13795 (2001); Chen-Hsiang et al., Bioinformatics 17 (Suppl. 1):S316-S322 (2001); Ramaswamy et al., Proc. Natl. Acad. Sci. USA 98:15149-15154 (2001)). Certain classifications of human breast cancers based on gene expression patterns have also been reported (Martin et al., Cancer Res. 60:2232-2238 (2000); West et al., Proc. Natl. Acad. Sci. USA 98:11462-11467 (2001); Sorlie et al., Proc. Natl. Acad Sci. USA 98:10869-10874 (2001); Yan et al., Cancer Res. 61:8375-8380 (2001)). However, these studies mostly focus on improving and refining the already established classification of various types of cancer, including breast cancer, and generally do not provide new insights into the relationships of the differentially expressed genes, and do not link the findings to treatment strategies in order to improve the clinical outcome of cancer therapy.

Although modem molecular biology and biochemistry have revealed hundreds of genes whose activities influence the behavior of tumor cells, state of their differentiation, and their sensitivity or resistance to certain therapeutic drugs, with a few exceptions, the status of these genes has not been exploited for the purpose of routinely making clinical decisions about drug treatments. One notable exception is the use of estrogen receptor (ER) protein expression in breast carcinomas to select patients to treatment with anti-estrogen drugs, such as tamoxifen. Another exceptional example is the use of ErbB2 (Her2) protein expression in breast carcinomas to select patients with the Her2 antagonist drug Herceptin® (Genentech, Inc., South San Francisco, CA).

Despite recent advances, the challenge of cancer treatment remains to target specific treatment regimens to pathogenically distinct tumor types, and ultimately personalize tumor treatment in order to maximize outcome. Hence, a need exists for tests that simultaneously provide predictive information about patient responses to the variety of treatment options. This is particularly true for breast cancer, the biology of which is poorly understood. It is clear that the classification of breast cancer into a few subgroups, such as the ErbB2 positive subgroup, and subgroups characterized by low to absent gene expression of the estrogen receptor (ER) and a few additional transcriptional factors (Perou et al., Nature 406:747-752 (2000)), does not reflect the cellular and molecular heterogeneity of breast cancer, and does not allow the design of treatment strategies maximizing patient response.

Breast cancer is the most common type of cancer among women in the United States and is the leading cause of cancer deaths among women ages 40 - 59. Therefore, there is a particularly great need for a clinically validated breast cancer test predictive of patient response to chemotherapy.

Ding et al. (2000) Proceedings of the American Association for Cancer Research 41, 404, propose that multidrug resistance in human cervical cells is associated with enhanced expression of the anti-apoptotic proteins BAG-1 and $BCL-X_L$ and reduced caspase-3 activity.

Kitada et al. (1998) Blood 91, 3379-89 disclose expression of apoptosis-regulating proteins in chronic lymphocytic leukaemia and correlations with *in vitro* and *in vivo* chemoresponses.

WO 2004/111603 discloses gene expression markers for predicting response to chemotherapy.

Muss *et al.* (1994) disclose *c-erb*B-2 expression and response to adjuvant therapy in women with node-positive early breast cancer (Muss et al. (1994) N. Eng. J. Med. 330, 1260-6).

Sjöström *et al.* (2002) disclose a study of the predictive value of bc1-2, bax, bc1-xL, bag-1, fas, and fasL for chemotherapy response in advanced breast cancer (Sjöström et al. (2002) Clin. Cancer Res. 8, 811-6).

Summary of the Invention

According to an aspect of the present invention, there is provided a method for determining whether a level of an RNA transcript, is associated with a beneficial response to chemotherapy treatment comprising an anthracycline and a taxane as specified in claim 1.

The breast cancer can, for example, be invasive breast cancer, or stage II or stage III breast cancer.

In a particular embodiment, the chemotherapy is adjuvant chemotherapy.

In another embodiment, the chemotherapy is neoadjuvant chemotherapy.

The chemotherapy may, for example, comprise the administration of docetaxel and/or paclitaxel, and/or doxorubicin.

The biological sample may be e.g. a tissue sample comprising cancer cells, where the tissue can be fixed, paraffin-embedded, or fresh, or frozen.

The expression level of said prognostic RNA transcript or transcripts can be determined, for example, by RT-PCR or any other PCR-based method, immunohistochemistry, proteomics techniques, or any other methods known in the art, or their combination.

Brief Description of the Drawings

Figure 1 shows the relationship between recurrence score (RS) and likelihood of patient response to chemotherapy, based on results from a clinical trial with pathologic complete response endpoint.

Table 1 shows a list of genes, the expression of which correlates, positively or negatively, with breast cancer response to adriamycin and taxane neoadjuvant chemotherapy. Results from a clinical trial with pathologic complete response endpoint. Statistical analysis utilized univarite generalized linear models with a probit link function.

Table 2 presents a list of genes, the expression of which predicts breast cancer response to chemotherapy. Results from a retrospective clinical trial. The table includes accession numbers for the genes, sequences for the forward and reverse primers (designated by "f" and "r", respectively) and probes (designated by "p") used for PCR amplification.

Table 3 shows the amplicon sequences used in PCR amplification of the indicated genes.

Detailed Description

A. Definitions

Unless defined otherwise, technical and scientific terms used herein have the same meaning as commonly understood by one of ordinary skill in the art to which this invention belongs. Singleton et al., Dictionary of Microbiology and Molecular Biology 2nd ed., J. Wiley& Sons (New York, NY 1994), and March, Advanced Organic Chemistry Reactions, Mechanisms and Structure 4th ed., John Wiley & Sons (New York, NY 1992), provide one skilled in the art with a general guide to many of the terms used in the present application.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

The term "microarray" refers to an ordered arrangement of hybridizable array elements, preferably polynucleotide probes, on a substrate.

The term "polynucleotide," when used in singular or plural, generally refers to any polyribonucleotide or polydeoxribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double-stranded DNAs. Oligonucleotides, such as single-stranded DNA probe oligonucleotides, are often synthesized by chemical methods, for example using automated oligonucleotide synthesizers that are commercially available. However, oligonucleotides can be made by a variety of other methods, including *in vitro* recombinant DNA-mediated techniques and by expression of DNAs in cells and organisms.

The terms "differentially expressed gene," "differential gene expression" and their synonyms, which are used interchangeably, refer to a gene whose expression is activated to a higher or lower level in a subject suffering from a disease, specifically cancer, such as breast cancer, relative to its expression in a normal or control subject. The terms also include genes whose expression is activated to a higher or lower level at different stages of the same disease. It is also understood that a differentially expressed gene may be either activated or inhibited at the nucleic acid level or protein level, or may be subject to alternative splicing to result in a different polypeptide product. Such differences may be evidenced by a change in mRNA levels, surface expression, secretion or other partitioning of a polypeptide, for example. Differential gene expression may include a comparison of expression between two or more genes or their gene products, or a comparison of the ratios of the expression between two or more genes or their gene products, or even a comparison of two differently processed products of the same gene, which differ between normal subjects and subjects suffering from a disease, specifically cancer, or between various stages of the same disease. Differential expression includes both quantitative, as well as qualitative, differences in the temporal or cellular expression pattern in a gene or its expression products among, for example, normal and diseased cells, or among cells which have undergone different disease events or disease stages. For the purpose of this invention, "differential gene expression" is considered to be present when there is at least an about two-fold, preferably at least about four-fold, more preferably at least about six-fold, most preferably at least about ten-fold difference between the expression of a given gene in normal and diseased subjects, or in various stages of disease development in a diseased subject.

The term "normalized" with regard to a gene transcript or a gene expression product refers to the level of the transcript or gene expression product relative to the mean levels of transcripts/products of a set of reference genes, wherein the reference genes are either selected based on their minimal variation across, patients, tissues or treatments ("housekeeping genes"), or the reference genes are the totality of tested genes. In the latter case, which is commonly referred to as "global normalization", it is important that the total number of tested genes be relatively large, preferably greater than 50. Specifically, the term 'normalized' with respect to an RNA transcript refers to the transcript level relative to the mean of transcript levels of a set of reference genes. More specifically, the mean level of an RNA transcript as measured by TaqMan® RT-PCR refers to the Ct value minus the mean Ct values of a set of reference gene transcripts.

The terms "expression threshold," and "defined expression threshold" are used interchangeably and refer to the level of a gene or gene product in question above which the gene or gene product serves as a predictive marker for patient response or resistance to a drug. The threshold typically is defined experimentally from clinical studies. The expression threshold can be selected either for maximum sensitivity (for example, to detect all responders to a drug), or for maximum selectivity (for example to detect only responders to a drug), or for minimum error.

The phrase "gene amplification" refers to a process by which multiple copies of a gene or gene fragment are formed in a particular cell or cell line. The duplicated region (a stretch of amplified DNA) is often referred to as "amplicon." Often, the amount of the messenger RNA (mRNA) produced, *i.e.*, the level of gene expression, also increases in the proportion to the number of copies made of the particular gene.

The term "prognosis" is used herein to refer to the prediction of the likelihood of cancer-attributable death or progression, including recurrence, metastatic spread, and drug resistance, of a neoplastic disease, such as breast cancer. The term "prediction" is used herein to refer to the likelihood that a patient will respond either favorably or unfavorably to a drug or set of drugs, and also the extent of those responses, or that a patient will survive, following surgical removal or the primary tumor and/or chemotherapy for a certain period of time without cancer recurrence. The predictive methods of the present invention can be used clinically to make treatment decisions by choosing the most appropriate treatment modalities for any particular patient. The predictive methods of the present invention are valuable tools in predicting if a patient is likely to respond favorably to a treatment regimen, such as surgical intervention, chemotherapy with a given drug or drug combination, and/or radiation therapy, or whether long-term survival of the patient, following surgery and/or termination of chemotherapy or other treatment modalities is likely.

The term "long-term" survival is used herein to refer to survival for at least 3 years, more preferably for at least 8 years, most preferably for at least 10 years following surgery or other treatment.

The term "tumor," as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, breast cancer, colon cancer, lung cancer, prostate cancer, hepatocellular cancer, gastric cancer, pancreatic cancer, cervical cancer, ovarian cancer, liver cancer, bladder cancer, cancer of the urinary tract, thyroid cancer, renal cancer, carcinoma, melanoma, and brain cancer.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, etc.

"Patient response" can be assessed using any endpoint indicating a benefit to the patient, including, without limitation, (1) inhibition, to some extent, of tumor growth, including slowing down and complete growth arrest; (2) reduction in the number of tumor cells; (3) reduction in tumor size; (4) inhibition (i.e., reduction, slowing down or complete stopping) of tumor cell infiltration into adjacent peripheral organs and/or tissues; (5) inhibition (i.e. reduction, slowing down or complete stopping) of metastasis; (6) enhancement of anti-tumor immune response, which may, but does not have to, result in the regression or rejection of the tumor; (7) relief, to some extent, of one or more symptoms associated with the tumor; (8) increase in the length of survival following treatment; and/or (9) decreased mortality at a given point of time following treatment.

"Neoadjuvant therapy" is adjunctive or adjuvant therapy given prior to the primary (main) therapy. Neoadjuvant therapy includes, for example, chemotherapy, radiation therapy, and hormone therapy. Thus, chemotherapy may be administered prior to surgery to shrink the tumor, so that surgery can be more effective, or, in the case of previously unoperable tumors, possible.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology. Wiley Interscience Publishers, (1995).

"Stringent conditions" or "high stringency conditions", as defined herein, typically: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5. x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

In the context of the present invention, reference to "at least one," "at least two," "at least five," etc. of the genes listed in any particular gene set means any one or any and all combinations of the genes listed.

B. Detailed Description

The practice of the present invention will employ, unless otherwise indicated, conventional techniques of molecular biology (including recombinant techniques), microbiology, cell biology, and biochemistry, which are within the skill of the art. Such techniques are explained fully in the literature, such as, "Molecular Clowning: A Laboratory Manual", 2nd edition (Sambrook et al., 1989); "Oligonucleotide Synthesis" (M.J. Gait, ed., 1984); "Animal Cell Culture" (R.I. Freshney, ed., 1987); "Methods in Enzymology" (Academic Press, Inc.); "Handbook of Experimental Immunology", 4th edition (D.M. Weir & C.C. Blackwell, eds., Blackwell Science Inc., 1987); "Gene Transfer Vectors for Mammalian Cells" (J.M. Miller & M.P. Calos, eds., 1987); "Current Protocols in Molecular Biology" (F.M. Ausubel et al., eds., 1987); and "PCR: The Polymerase Chain Reaction", (Mullis et al., eds., 1994).

1. *Gene Expression Profiling*

Methods of gene expression profiling include methods based on hybridization analysis of polynucleotides, methods based on sequencing of polynucleotides, and proteomics-based methods. The most commonly used methods known in the art for the quantification of mRNA expression in a sample include northern blotting and *in situ* hybridization (Parker & Barnes, Methods in Molecular Biology 106:247-283 (1999)); RNAse protection assays (Hod, Biotechniques 13:852-854 (1992)); and PCR-based methods, such as reverse transcription polymerase chain reaction (RT-PCR) (Weis et al., Trends in Genetics 8:263-264 (1992)). Alternatively, antibodies may be employed that can recognize specific duplexes, including DNA duplexes, RNA duplexes, and DNA-RNA hybrid duplexes or DNA-protein duplexes. Representative methods for sequencing-based gene expression analysis include Serial Analysis of Gene Expression (SAGE), and gene expression analysis by massively parallel signature sequencing (MPSS).

2. *PCR-based Gene Expression Profiling Methods*

a. *Reverse Transcriptase PCR (RT-PCR)*

One of the most sensitive and most flexible quantitative PCR-based gene expression profiling methods is RT-PCR, which can be used to compare mRNA levels in different sample populations, in normal and tumor tissues, with or without drug treatment, to characterize patterns of gene expression, to discriminate between closely related mRNAs, and to analyze RNA structure.

The first step is the isolation of mRNA from a target sample. The starting material is typically total RNA isolated from human tumors or tumor cell lines, and corresponding normal tissues or cell lines, respectively. Thus RNA can be isolated from a variety of primary tumors, including breast, lung, colon, prostate, brain, liver, kidney, pancreas, spleen, thymus, testis, ovary, uterus, etc., tumor, or tumor cell lines, with pooled DNA from healthy donors. If the source of mRNA is a primary tumor, mRNA can be extracted, for example, from frozen or archived paraffin-embedded and fixed (e.g. formalin-fixed) tissue samples.

General methods for mRNA extraction are well known in the art and are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andress et al., BioTechniques 18:42044 (1995). In particular, RNA isolation can be performed using purification kit, buffer set and protease from commercial manufacturers, such as Qiagen, according to the manufacturer's instructions. For example, total RNA from cells in culture can be isolated using Qiagen RNeasy mini-columns. Other commercially available RNA isolation kits include MasterPure™ Complete DNA and RNA Purification Kit (EPICENTRE®, Madison, WI), and Paraffin Block RNA Isolation Kit (Ambion, Inc.). Total RNA from tissue samples can be isolated using RNA Stat-60 (Tel-Test). RNA prepared from tumor can be isolated, for example, by cesium chloride density gradient centrifugation.

As RNA cannot serve as a template for PCR, the first step in gene expression profiling by RT-PCR is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan® PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan® RT-PCR can be performed using commercially available equipment, such as, for example, ABI PRISM 7700™ Sequence Detection System™ (Perkin-Elmer-Applied Biosystems, Foster City, CA, USA), or Lightcycler (Roche Molecular Biochemicals, Mannheim, Germany). In a preferred embodiment, the 5' nuclease procedure is run on a real-time quantitative PCR device such as the ABI PRISM 7700™ Sequence Detection System™. The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle ($C_t$).

To minimize errors and the effect of sample-to-sample variation, RT-PCR is usually performed using a reference RNA which ideally is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPD) and β-actin (ACTB).

A more recent variation of the RT-PCR technique is the real time quantitative PCR, which measures PCR product accumulation through a dual-labeled fluorigenic probe (i.e., TaqMan® probe). Real time PCR is compatible both with quantitative competitive PCR, where internal competitor for each target sequence is used for normalization, and with quantitative comparative PCR using a normalization gene contained within the sample, or a housekeeping gene for RT-PCR. For further details see, e.g. Held et al., Genome Research 6:986-994 (1996).

b. *MassARRAY System*

In the MassARRAY-based gene expression profiling method, developed by Sequenom, Inc. (San Diego, CA) following the isolation of RNA and reverse transcription, the obtained cDNA is spiked with a synthetic DNA molecule (competitor), which matches the targeted cDNA region in all positions, except a single base, and serves as an internal standard. The cDNA/competitor mixture is PCR amplified and is subjected to a post-PCR shrimp alkaline phosphatase (SAP) enzyme treatment, which results in the dephosphorylation of the remaining nucleotides. After inactivation of the alkaline phosphatase, the PCR products from the competitor and cDNA are subjected to primer extension, which generates distinct mass signals for the competitor- and cDNA-derives PCR products. After purification, these products are dispensed on a chip array, which is pre-loaded with components needed for analysis with matrix-assisted laser desorption ionization time-of-flight mass spectrometry, (MALDI-TOF MS) analysis. The cDNA present in the reaction is then quantified by analyzing the ratios of the peak areas in the mass spectrum generated. For further details see, e.g. Ding and Cantor, Proc. Natl. Acad Sci. USA 100:3059-3064 (2003).

c. *Other PCR-based Methods*

Further PCR-based techniques include, for example, differential display (Liang and Pardee, Science 257:967-971 (1992)); amplified fragment length polymorphism (iAFLP) (Kawamoto et al., Genome Res. 12:1305-1312 (1999)); BeadArray™ technology (Illumina, San Diego, CA; Oliphant et al., Discovery of Markers for Disease (Supplement to Biotechniques), June 2002; Ferguson et al., Analytical Chemistry 72:5618 (2000)); BeadsArray for Detection of Gene Expression (BADGE), using the commercially available Luminex$^{100}$ LabMAP system and multiple color-coded microspheres (Luminex Corp., Austin, TX) in a rapid assay for gene expression (Yang et al., Genome Res. 11:1888-1898 (2001)); and high coverage expression profiling (HiCEP)analysis (Fukumura et al., Nucl. Acids. Res. 31(16) e94 (2003)).

3. *Microarrays*

Differential gene expression can also be identified, or confirmed using the microarray technique. Thus, the expression profile of breast cancer-associated genes can be measured in either fresh or paraffin-embedded tumor tissue, using microarray technology. In this method, polynucleotide sequences of interest (including cDNAs and oligonucleotides) are plated, or arrayed, on a microchip substrate. The arrayed sequences are then hybridized with specific DNA probes from cells or tissues of interest. Just as in the RT-PCR method, the source of mRNA typically is total RNA isolated from human tumors or tumor cell lines, and corresponding normal tissues or cell lines. Thus RNA can be isolated from a variety of primary tumors or tumor cell lines. If the source of mRNA is a primary tumor, mRNA can be extracted, for example, from frozen or archived paraffin-embedded and fixed (e.g. formalin-fixed) tissue samples, which are routinely prepared and preserved in everyday clinical practice.

In a specific embodiment of the microarray technique, PCR amplified inserts of cDNA clones are applied to a substrate in a dense array. Preferably at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pairwise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93(2):106-149 (1996)). Microarray analysis can be performed by commercially available equipment, following manufacturer's protocols, such as by using the Affymetrix GenChip technology, or Incyte's microarray technology.

The development of microarray methods for large-scale analysis of gene expression makes it possible to search systematically for molecular markers of cancer classification and outcome prediction in a variety of tumor types.

4. *Serial Analysis of Gene Expression (SAGE)*

Serial analysis of gene expression (SAGE) is a method that allows the simultaneous and quantitative analysis of a large number of gene transcripts, without the need of providing an individual hybridization probe for each transcript. First, a short sequence tag (about 10-14 bp) is generated that contains sufficient information to uniquely identify a transcript, provided that the tag is obtained from a unique position within each transcript. Then, many transcripts are linked together to form long serial molecules, that can be sequenced, revealing the identity of the multiple tags simultaneously. The expression pattern of any population of transcripts can be quantitatively evaluated by determining the abundance of individual tags, and identifying the gene corresponding to each tag. For more details see, e.g. Velculescu et al., Science 270:484-487 (1995); and Velculescu et al., Cell 88:243-51 (1997).

*5. Gene Expression Analysis by Massively Parallel Signature Sequencing (MPSS)*

This method, described by Brenner et al., Nature Biotechnology 18:630-634 (2000), is a sequencing approach that combines non-gel-based signature sequencing with *in vitro* cloning of millions of templates on separate 5 $\mu$m diameter microbeads. First, a microbead library of DNA templates is constructed by *in vitro* cloning. This is followed by the assembly of a planar array of the template-containing microbeads in a flow cell at a high density (typically greater than 3 x $10^6$ microbeads/$cm^2$). The free ends of the cloned templates on each microbead are analyzed simultaneously, using a fluorescence-based signature sequencing method that does not require DNA fragment separation. This method has been shown to simultaneously and accurately provide, in a single operation, hundreds of thousands of gene signature sequences from a yeast cDNA library.

6. *Immunohistochemistry*

Immunohistochemistry methods are also suitable for detecting the expression levels of the prognostic markers of the present invention. Thus, antibodies or antisera, preferably polyclonal antisera, and most preferably monoclonal antibodies specific for each marker are used to detect expression. The antibodies can be detected by direct labeling of the antibodies themselves, for example, with radioactive labels, fluorescent labels, hapten labels such as, biotin, or an enzyme such as horse radish peroxidase or alkaline phosphatase. Alternatively, unlabeled primary antibody is used in conjunction with a labeled secondary antibody, comprising antisera, polyclonal antisera or a monoclonal antibody specific for the primary antibody. Immunohistochemistry protocols and kits are well known in the art and are commercially available.

7. *Proteomics*

The term "proteome" is defined as the totality of the proteins present in a sample (e.g. tissue, organism, or cell culture) at a certain point of time. Proteomics includes, among other things, study of the global changes of protein expression in a sample (also referred to as "expression proteomics"). Proteomics typically includes the following steps: (1) separation of individual proteins in a sample by 2-D gel electrophoresis (2-D PAGE); (2) identification of the individual proteins recovered from the gel, e.g. my mass spectrometry or N-terminal sequencing, and (3) analysis of the data using bioinformatics. Proteomics methods are valuable supplements to other methods of gene expression profiling, and can be used, alone or in combination with other methods, to detect the products of the prognostic markers of the present invention.

8. *General Description of mRNA Isolation, Purification and Amplification*

The steps of a representative protocol for profiling gene expression using fixed, paraffin-embedded tissues as the RNA source, including mRNA isolation, purification, primer extension and amplification are given in various published journal articles (for example: T.E. Godfrey et al. J. Molec. Diagnostics 2: 84-91 [2000]; K. Specht et al., Am. J. Pathol. 158: 419-29 [2001]). Briefly, a representative process starts with cutting about 10 μm thick sections of paraffin-embedded tumor tissue samples. The RNA is then extracted, and protein and DNA are removed. After analysis of the RNA concentration, RNA repair and/or amplification steps may be included, if necessary, and RNA is reverse transcribed using gene specific promoters followed by RT-PCR. Finally, the data are analyzed to identify the best treatment option(s) available to the patient on the basis of the characteristic gene expression pattern identified in the tumor sample examined.

9. *Cancer Chemotherapy*

Chemotherapeutic agents used in cancer treatment can be divided into several groups, depending on their mechanism of action. Some chemotherapeutic agents directly damage DNA and RNA. By disrupting replication of the DNA such chemotherapeutics either completely halt replication, or result in the production of nonsense DNA or RNA. This category includes, for example, cisplatin (Platinol®), daunorubicin (Cerubidine®), doxorubicin (Adriamycin®), and etoposide (VePesid®). Another group of cancer chemotherapeutic agents interfere with the formation of nucleotides or deoxyribonucleotides, so that RNA synthesis and cell replication is blocked. Examples of drugs in this class include methotrexate (Abitrexate®), mercaptopurine (Purinethol®), fluorouracil (Adrucil®), and hydroxyurea (Hydrea®). A third class of chemotherapeutic agents effects the synthesis or breakdown of mitotic spindles, and, as a result, interrupt cell division. Examples of drugs in this class include Vinblastine (Velban®), Vincristine (Oncovin®) and taxenes, such as, Pacitaxel (Taxol®), and Tocetaxel (Taxotere®) Tocetaxel is currently approved in the United States to treat patients with locally advanced or metastatic breast cancer after failure of prior chemotherapy, and patients with locally advanced or metastatic non-small cell lung cancer after failure of prior platinum-based chemotherapy.

A common problem with chemotherapy is the high toxicity of chemotherapeutic agents, such as anthracyclines and taxenes, which limits the clinical benefits of this treatment approach.

Most patients receive chemotherapy immediately following surgical removal of tumor. This approach is commonly referred to as adjuvant therapy. However, chemotherapy can be administered also before surgery, as so called neoadjuvant treatment. Although the use of neoadjuvant chemotherapy originates from the treatment of advanced and inoperable breast cancer, it has gained acceptance in the treatment of other types of cancers as well. The efficacy of neoadjuvant chemotherapy has been tested in several clinical trials. In the multi-center National Surgical Adjuvant Breast and Bowel Project B-18 (NSAB B-18) trial (Fisher et al., J. Clin. Oncology 15:2002-2004 (1997); Fisher et al., J. Clin. Oncology 16:2672-2685 (1998)) neoadjuvant therapy was performed with a combination of adriamycin and cyclophosphamide ("AC regimen"). In another clinical trial, neoadjuvant therapy was administered using a combination of 5-fluorouracil, epirubicin and cyclophosphamide ("FEC regimen") (van Der Hage et al., J. Clin. Oncol. 19:4224-4237 (2001)). Newer clinical trials have also used taxane-containing neoadjuvant treatment regiments. See, e.g. Holmes et al., J. Natl. Cancer Inst. 83:1797-1805 (1991) and Moliterni et al., Seminars in Oncology, 24:S17-10-S-17-14 (1999). For further information about neoadjuvant chemotherapy for breast cancer see, Cleator et al., Endocrine-Related Cancer 9:183-195 (2002).

10. *Cancer Gene Set, Assayed Gene Subsequences, and Clinical Application of Gene Expression Data*

An important aspect of the present invention is to use the measured expression of certain genes by breast cancer tissue to provide prognostic information. For this purpose it is necessary to correct for (normalize away) differences in the amount of RNA assayed, variability in the quality of the RNA used, and other factors, such as machine and operator differences. Therefore, the assay typically measures and incorporates the use of reference RNAs, including those transcribed from well-known housekeeping genes, such as GAPD and ACTB. A precise method for normalizing gene expression data is given in "User Bulletin #2" for the ABI PRISM 7700 Sequence Detection System (Applied Biosystems; 1997). Alternatively, normalization can be based on the mean or median signal (Ct) of all of the assayed genes or a large subset thereof (global normalization approach). In the study described in the following Example, a so called central normalization strategy was used, which utilized a subset of the screened genes, selected based on lack of correlation with clinical outcome, for normalization.

11. *Recurrence and Response to Therapy Scores and their Applications*

Copending application Serial No. 60/486,302, filed on July 10, 2003, describes an algorithm-based prognostic test for determining the likelihood of cancer recurrence and/or the likelihood that a patient responds well to a treatment modality. Features of the algorithm that distinguish it from other cancer prognostic methods include: 1) a unique set of test mRNAs (or the corresponding gene expression products) used to determine recurrence likelihood, 2) certain weights used to combine the expression data into a formula, and 3) thresholds used to divide patients into groups of different levels of risk, such as low, medium, and high risk groups. The algorithm yields a numerical recurrence score (RS) or, if patient response to treatment is assessed, response to therapy score (RTS).

The test requires a laboratory assay to measure the levels of the specified mRNAs or their expression products, but can utilize very small amounts of either fresh tissue, or frozen tissue or fixed, paraffin-embedded tumor biopsy specimens that have already been necessarily collected from patients and archived. Thus, the test can be noninvasive. It is also compatible with several different methods of tumor tissue harvest, for example, via core biopsy or fine needle aspiration.

According to the method, cancer recurrence score (RS) is determined by:

(a) subjecting a biological sample comprising cancer cells obtained from said subject to gene or protein expression profiling;
(b) quantifying the expression level of multiple individual genes [i.e., levels of mRNAs or proteins] so as to determine an expression value for each gene;
(c) creating subsets of the gene expression values, each subset comprising expression values for genes linked by a cancer-related biological function and/or by co-expression;
(d) multiplying the expression level of each gene within a subset by a coefficient reflecting its relative contribution to cancer recurrence or response to therapy within said subset and adding the products of multiplication to yield a term for said subset;
(e) multiplying the term of each subset by a factor reflecting its contribution to cancer recurrence or response to therapy; and
(f) producing the sum of terms for each subset multiplied by said factor to produce a recurrence score (RS) or a response to therapy (RTS) score,

wherein the contribution of each subset which does not show a linear correlation with cancer recurrence or response to therapy is included only above a predetermined threshold level, and
wherein the subsets in which increased expression of the specified genes reduce risk of cancer recurrence are assigned a negative value, and the subsets in which expression of the specified genes increase risk of cancer recurrence are assigned a positive value.

In a particular embodiment, RS is determined by:

(a) determining the expression levels of GRB7, HER2, EstR1, PR, Bc12, CEGP1, SURV, Ki.67, MYBL2, CCNB1, STK15, CTSL2, STMY3, CD68, GSTM1, and BAG1, or their expression products, in a biological sample containing tumor cells obtained from said subject; and
(b) calculating the recurrence score (RS) by the following equation: RS = (0.23 to 0.70) x GRB7axisthresh - (0.17 to 0.51) x ERaxis + (0.53 to 1.56) x prolifaxisthresh + (0.07 to 0.21) x invasionaxis + (0.03 to 0.15) x CD68 - (0.04 to 0.25) x GSTM1 - (0.05 to 0.22) x BAG1
wherein

(i) GRB7 axis = (0.45 to 1.35) x GRB7 + (0.05 to 0.15) x HER2;
(ii) if GRB7 axis < -2, then GRB7 axis thresh = -2, and if GRB7 axis ≥ -2, then GRB7 axis thresh = GRB7 axis;
(iii) ER axis = (Est1 + PR + Bc12 + CEGP1)/4;
(iv) prolifaxis = (SURV + Ki.67 + MYBL2 + CCNB1 + STK15)/5;
(v) if prolifaxis < -3.5, then prolifaxisthresh = -3.5, if prolifaxis ≥ -3.5, then prolifaxishresh = prolifaxis; and
(vi) invasionaxis = (CTSL2 + STMY3)/2,

wherein the terms for all individual genes for which ranges are not specifically shown can vary between about 0.5 and 1.5, and wherein a higher RS represents an increased likelihood of cancer recurrence.

Further details of the invention will be described in the following non-limiting Example.

## Example

A Retrospective Study of Neoadjuvant Chemotherapy in Invasive Breast

Cancer: Gene Expression Profiling of Paraffin-Embedded Core Biopsy Tissue

This was a collaborative study involving Genomic Health, Inc., (Redwood City California), and Institute Tumori, Milan, Italy. The primary objective of the study was to explore the correlation between pre-treatment molecular profiles and pathologic complete response (pCR) to neoadjuvant chemotherapy in locally advanced breast cancer.

*Patient inclusion criteria:*

Histologic diagnosis of invasive breast cancer (date of surgery 1998-2002); diagnosis of locally advanced breast cancer defined by skin infiltration and or N2 axillary status and or homolateral supraclavicular positive nodes; core biopsy, neoadjuvant chemotherapy and surgical resection performed at Istituto Nazionale Tumori, Milan; signed informed consent that the biological material obtained for histological diagnosis or diagnostic procedures would be used for research; and histopathologic assessment indicating adequate amounts of tumor tissue for inclusion in this research study.

*Exclusion criteria:*

Distant metastases; no tumor block available from initial core biopsy or from the surgical resection; or no tumor or very little tumor (<5% of the overall tissue on the slide) in block as assessed by examination of the H&E slide by the Pathologist.

*Study design*

Eighty-nine evaluable patients (from a set of 96 clinically evaluable patients) were identified and studied. The levels of 384 mRNA species were measured by RT-PCR, representing products of candidate cancer-related genes that were selected from the biomedical research literature. Only one gene was lost due to inadequate signal.

Patient characteristics were as follows: Mean age: 50 years; Tumor grades: 24% Well, 55% Moderate, and 21 % Poor; Sixty-three % of patients were ER positive {by immunohistochemistry}; Seventy % of patients had positive lymph nodes.

All patients were given primary neoadjuvant chemotherapy: Doxorubicin plus Taxol 3weeks/3 cycles followed by Taxol® (paclitaxel) 1week/12 cycles. Surgical removal of the tumor followed completion of chemotherapy. Core tumor biopsy specimens were taken prior to start of chemotherapy, and served as the source of RNA for the RT-PCR assay.

*Materials and Methods*

Fixed paraffin-embedded (FPE) tumor tissue from biopsy was obtained prior to and after chemotherapy. Core biopsies were taken prior to chemotherapy. In that case, the pathologist selected the most representative primary tumor block, and submitted nine 10 micron sections for RNA analysis. Specifically, a total of 9 sections (10 microns in thickness each) were prepared and placed in three Costar Brand Microcentrifuge Tubes (Polypropylene, 1.7 mL tubes, clear; 3 sections in each tube) and pooled.

Messenger RNA was extracted using the *MasterPure™ RNA Purification Kit* (Epicentre Technologies) and quantified by the RiboGreen® fluorescence method (Molecular probes). Molecular assays of quantitative gene expression were performed by RT-PCR, using the ABI PRISM 7900™ Sequence Detection System™ (Perkin-Elmer-Applied Biosystems, Foster City, CA, USA). ABI PRISM 7900™ consists of a thermocycler, laser, charge-coupled device (CCD), camera and computer. The system amplifies samples in a 384-well format on a thermocycler. During amplification, laser-induced fluorescent signal is collected in real-time for all 384 wells, and detected at the CCD. The system includes software for running the instrument and for analyzing the data.

*Analysis and Results*

Tumor tissue was analyzed for 384 genes. The threshold cycle ($C_T$) values for each patient were normalized based on the median of a subset of the screened genes for that particular patient, selected based on lack of correlation with clinical outcome (central normalization strategy). Patient beneficial response to chemotherapy was defined as pathologic complete response (pCR). Patients were formally assessed for response at the completion of all chemotherapy.

A clinical complete response (cCR) requires complete disappearance of all clinically detectable disease, either by physical examination or diagnostic breast imaging.

A pathologic complete response (pCR) requires absence of residual breast cancer on histologic examination of biopsied breast tissue, lumpectomy or mastectomy specimens following primary chemotherapy. Residual ductal carcinoma *in situ* (DCTS) may be present.

Residual cancer in regional nodes may not be present. Of the 89 evaluable patients 11 (12%) had a pathologic complete response (pCR). Seven of these patients were ER negative.

A partial clinical response was defined as a ≥ 50% decrease in tumor area (sum of the products of the longest perpendicular diameters) or a ≥ 50% decrease in the sum of the products of the longest perpendicular diameters of multiple lesions in the breast and axilla. No area of disease may increase by > 25% and no new lesions may appear.

Analysis was performed by comparing the relationship between normalized gene expression and the binary outcomes of pCR or no pCR. Univariate generalized models were used with probit or logit link functions. See, e.g. Van K. Borooah, LOGIT and PROBIT, Ordered Multinominal Models, Sage University Paper, 2002.

Table 1 presents pathologic response correlations with gene expression, and lists the 86 genes for which the p-value for the differences between the groups was <0.1. The second column (with the heading "Direction") denotes whether increased expression correlates with decreasing or increasing likelihood of response to chemotherapy. The statistical significance of the predictive value for each gene is given by P-value (right hand column)

| | | Probit Link | | |
|---|---|---|---|---|
| **Gene** | **Direction** | **Intercept** | **Slope** | **P-value** |
| TBP | Decreasing | 0.0575 | 2.4354 | 0.0000 |
| ILT.2 | Increasing | 0.5273 | -0.9489 | 0.0003 |
| ABCC5 | Decreasing | 0.9872 | 0.8181 | 0.0003 |
| CD18 | Increasing | 3.4735 | -1.0787 | 0.0007 |
| GATA3 | Decreasing | 0.6175 | 0.2975 | 0.0008 |
| DICER1 | Decreasing | -0.9149 | 1.4875 | 0.0013 |
| MSH3 | Decreasing | 2.6875 | 0.9270 | 0.0013 |
| GBP1 | Increasing | 1.7649 | -0.5410 | 0.0014 |
| IRS1 | Decreasing | 1.3576 | 0.5214 | 0.0016 |
| CD3z | Increasing | 0.1567 | -0.5162 | 0.0018 |
| Fasl | Increasing | -0.6351 | -0.4050 | 0.0019 |
| TUBB | Decreasing | 1.2745 | 0.8267 | 0.0025 |
| BAD | Decreasing | 0.9993 | 1.1325 | 0.0033 |
| ERCC1 | Decreasing | 0.0327 | 1.0784 | 0.0039 |
| MCM6 | Increasing | 0.1371 | -0.8008 | 0.0052 |
| PR | Decreasing | 1.6079 | 0.1764 | 0.0054 |
| APC | Decreasing | 0.7264 | 1.0972 | 0.0061 |
| GGPS1 | Decreasing | 1.0906 | 0.8124 | 0.0062 |
| KRT18 | Decreasing | -0.8029 | 0.4506 | 0.0063 |
| ESRRG | Decreasing | 2.0198 | 0.2262 | 0.0063 |
| E2F1 | Increasing | 0.2188 | -0.5277 | 0.0068 |
| AKT2 | Decreasing | -1.3566 | 1.1902 | 0.0074 |
| A.Catenin | Decreasing | -0.6859 | 0.9279 | 0.0079 |
| CEGP1 | Decreasing | 1.3355 | 0.1875 | 0.0091 |
| NPD009 | Decreasing | 1.3996 | 0.2971 | 0.0092 |
| MAPK14 | Decreasing | 2.6253 | 1.6007 | 0.0093 |
| RUNX1 | Decreasing | -0.4138 | 0.7214 | 0.0103 |
| ID2 | Increasing | 1.7326 | -0.7032 | 0.0104 |
| G.Catenin | Decreasing | -0.1221 | 0.5954 | 0.0110 |

(continued)

| | | Probit Link | | |
|---|---|---|---|---|
| Gene | Direction | Intercept | Slope | P-value |
| FBXO5 | Increasing | 0.3421 | -0.4935 | 0.0110 |
| FHIT | Decreasing | 1.9966 | 0.4989 | 0.0113 |
| MTA1 | Decreasing | 0.3127 | 0.6069 | 0.0133 |
| ERBB4 | Decreasing | 1.4591 | 0.1436 | 0.0135 |
| FUS | Decreasing | -0.6150 | 0.9415 | 0.0137 |
| BBC3 | Decreasing | 2.4796 | 0.6495 | 0.0138 |
| IGF1R | Decreasing | 1.1998 | 0.3116 | 0.0147 |
| CD9 | Decreasing | -0.9292 | 0.5747 | 0.0156 |
| TP53BP1 | Decreasing | 1.4325 | 0.8122 | 0.0169 |
| MUC1 | Decreasing | 0.8881 | 0.2140 | 0.0175 |
| IGFBP5 | Decreasing | -0.6180 | 0.4880 | 0.0181 |
| rhoC | Decreasing | -0.1726 | 0.6860 | 0.0184 |
| RALBP1 | Decreasing | 0.2383 | 0.9509 | 0.0185 |
| CDC20 | Increasing | 1.3204 | -0.4390 | 0.0186 |
| STAT3 | Decreasing | -0.9763 | 0.7023 | 0.0194 |
| ERK1 | Decreasing | 0.8577 | 0.6496 | 0.0198 |
| HLA.DPB1 | Increasing | 3.6300 | -0.6035 | 0.0202 |
| SGCB | Decreasing | 0.6171 | 0.7823 | 0.0208 |
| CGA | Increasing | 0.0168 | -0.1450 | 0.0209 |
| DHPS | Decreasing | 0.2957 | 0.7840 | 0.0216 |
| MGMT | Decreasing | 0.9238 | 0.6876 | 0.0226 |
| CRIP2 | Decreasing | 0.5524 | 0.4394 | 0.0230 |
| MMP12 | Increasing | 0.4208 | -0.2419 | 0.0231 |
| ErbB3 | Decreasing | 0.9438 | 0.2798 | 0.0233 |
| RAP1GDS1 | Decreasing | 0.2617 | 0.7672 | 0.0235 |
| CDC25B | Increasing | 1.6965 | -0.5356 | 0.0264 |
| IL6 | Increasing | 0.0592 | -0.2388 | 0.0272 |
| CCND1 | Decreasing | 0.2260 | 0.2992 | 0.0272 |
| CYBA | Increasing | 2.6493 | -0.5175 | 0.0287 |
| PRKCD | Decreasing | 0.2125 | 0.6745 | 0.0291 |
| DR4 | Increasing | 0.3039 | -0.5321 | 0.0316 |
| Hepsin | Decreasing | 1.9211 | 0.1873 | 0.0318 |
| CRABP1 | Increasing | 1.0309 | -0.1287 | 0.0320 |
| AK055699 | Decreasing | 2.0442 | 0.1765 | 0.0343 |
| Contig.51037 | Increasing | 0.7857 | -0.1131 | 0.0346 |
| VCAM1 | Increasing | 1.1866 | -0.3560 | 0.0346 |
| FYN | Increasing | 1.5502 | -0.5624 | 0.0359 |
| GRB7 | Increasing | 1.3592 | -0.1646 | 0.0375 |
| AKAP.2 | Increasing | 1.7946 | -0.7008 | 0.0382 |
| RASSF1 | Increasing | 1.1972 | -0.0390 | 0.0384 |
| MCP1 | Increasing | 1.3700 | -0.3805 | 0.0388 |
| ZNF38 | Decreasing | 1.7957 | 0.4993 | 0.0395 |
| MCM2 | Increasing | 1.0574 | -0.4695 | 0.0426 |
| GBP2 | Increasing | 1.4095 | -0.4559 | 0.0439 |
| SEMA3F | Decreasing | 1.2706 | 0.3725 | 0.0455 |
| CD31 | Increasing | 1.9913 | -0.5955 | 0.0459 |
| COL1A1 | Decreasing | -1.9861 | 0.3812 | 0.0466 |
| ER2 | Increasing | -0.5204 | -0.2617 | 0.0471 |

(continued)

| | | Probit Link | | |
|---|---|---|---|---|
| Gene | Direction | Intercept | Slope | P-value |
| BAG1 | Decreasing | 0.6731 | 0.5070 | 0.0472 |
| AKT1 | Decreasing | -0.4467 | 0.5768 | 0.0480 |
| COL1A2 | Decreasing | -1.0233 | 0.3804 | 0.0490 |
| STAT1 | Increasing | 1.9447 | -0.4062 | 0.0498 |
| Wnt.5a | Decreasing | 2.2244 | 0.2983 | 0.0518 |
| PTPD1 | Decreasing | 1.2950 | 0.4834 | 0.0552 |
| RAB6C | Decreasing | 0.4841 | 0.5635 | 0.0717 |
| TK1 | Increasing | 0.6127 | -0.3625 | 0.0886 |
| Bcl2 | Decreasing | 1.1459 | 0.2509 | 0.0959 |

Based on the data set forth in Table 1, increased expression of the following genes correlates with increased likelihood of complete pathologic response to treatment: ILT.2; CD18; GBP1; CD3z; fasl; MCM6; E2F1; ID2; FBXO5; CDC20; HLA.DPB1; CGA; MMP12; CDC25B; IL6; CYBA; DR4; CRABP1; Contig.51037; VCAM1; FYN; GRB7; AKAP.2; RASSF1; MCP1; MCM2; GBP2; CD31; ER2; STAT1; TK1; while increased expression of the following genes correlates with decreased likelihood of complete pathologic response to treatment: TBP; ABC5; GATA3; DICER1; MSH3; IRS1; TUBB; BAD; ERCC1; PR; APC; GGPS1; KRT18; ESRRG; AKT2; A.Catenin; CEGP1; NPD009; MAPK14; RUNX1; G.Catenin; FHIT; MTA1; ErbB4; FUS; BBC3; IGF1R; CD9; TP53BP1; MUC1; IGFBP5; rhoC; RALBP1; STAT3; ERK1; SGCB; DHPS; MGMT; CRISP2; ErbB3; RAP1GDS1; CCND 1; PRKCD; Hepsin, AK055699; ZNF38; SEMA3F; COL1A1; BAG1; AKT1; COL1A2; Wnt.5a; PTPD1; RAB6C; Bcl2.

The relationship between the recurrence risk algorithm (described in copending U.S. application Serial No. 60/486,302) and pCR was also investigated. The algorithm incorporates the measured levels of 21 mRNA species. Sixteen mRNAs (named below) were candidate clinical markers and the remaining 5 (ACTB, GAPD, GUSB, RPLPO, and TFRC) were reference genes. Reference-normalized expression measurements range from 0 to 15, where a one unit increase reflects a 2-fold increase in RNA.

The Recurrence Score (RS) is calculated from the quantitative expression of four sets of marker genes (an estrogen receptor group of 4 genes-ESR1, PGR, BCL2, and SCUBE2; a proliferation set of 5 genes-MKI67, MYBL2, BIRC5, CCNB1, and STK6; a HER2 set of 2 genes-ERBB2 and GRB7, an invasion group of 2 genes-MMP11 and CTSL2) and 3 other individual genes-GSTM1, BAG1, and CD68.

Although the genes and the multiplication factors used in the equation may vary, in a typical embodiment, the following equation may be used to calculate RS:

$$\text{RS (range, 0 – 100)} = \begin{aligned} &+ 0.47 \times \text{HER2 Group Score} \\ &- 0.34 \times \text{ER Group Score} \\ &+ 1.04 \times \text{Proliferation Group Score} \\ &+ 0.10 \times \text{Invasion Group Score} \\ &+ 0.05 \times \text{CD68} \\ &- 0.08 \times \text{GSTM1} \\ &- 0.07 \times \text{BAG1} \end{aligned}$$

Application of this algorithm to study clinical and gene expression data sets yields a continuous curve relating RS to pCR values, as shown in Figure 1. Examination of these data shows that patients with RS > 50 are in the upper 50 percentile of patients in terms of likelihood of response to chemotherapy, and that patients with RS < 35 are in the lower 50 percentile of patients in terms of likelihood of response to chemotherapy.

While the invention has been described with emphasis upon certain specific embodiments, it is be apparent to those skilled in the art that variations and modification in the specific methods and techniques are possible. Accordingly, this invention includes all modifications encompassed within the spirit and scope of the invention as defined by the following claims.

**TABLE 2**

| | | | |
|---|---|---|---|
| A-Catenin | NM_00190 S21381A, Cate.f2 | CGTTCCGATCCTCTATACTGCAT | 23 |
| A-Catenin | NM_00190 S2139/A-Cate.r2 | AGGTCCCTGTTGGCCTTATAGG | 22 |
| A-Catenin | NM_00190 S4725/A'-Cate.p2 | ATGCCTACAGCACCCTGATGTCGCA | 25 |
| ABCC5 | NM_00568 S5605/ABCC5.f1 | TGCAGACTGTACCATGCTGA | 20 |
| ABCC5 | NM_00568 S5606/ABCC5.r1 | GGCCAGCACCATAATCCTAT | 20 |
| ABCC5 | NM_00568 S5607/ABCC5.p1 | CTGCACACGGTTCTAGGCTCCG | 22 |
| AK055699 | AK055699 S2097/AK0556.f1 | CTGCATGTGATTGAATAAGAAACAAGA | 27 |
| AK055699 | AK055699 S2098/AK0556.r1 | TGTGGACCTGATCCCTGTACAC | 22 |
| AK055699 | AK055699 S5057/AK0556.p1 | TGACCACACCAAAGCCTCCCTGG | 23 |
| AKAP-2 | NM_00720 S13741AKAP-2.f1 | ACGAATTGTCGGTGAGGTCT | 20 |
| AKAP-2 | NM_00720 S1375/AKAP-2.r1 | GTCCATGCTGAAATCATTGG | 20 |
| AKAP-2 | NM_00720 S4934/AKAP-2.p1 | CAGGATACCACAGTCCTGGAGACCC | 25 |
| AKT1 | NM_00516 S0010/AKT1.f3 | CGCTTCTATGGCGCTGAGAT | 20 |
| AKT1 | NM_00516 S0012IAKT1.r3 | TCCCGGTACACCACGTTCTT | 20 |
| AKT1 | NM_00516 S4776/AKT1.p3 | CAGCCCTGGACTACCTGCACTCGG | 24 |
| AKT2 | NM_00162 S0828/AKT2.f3 | TCCTGCCACCCTTCAAACC | 19 |
| AKT2 | NM_00162 S0829/AKT2.r3 | GGCGGTAAATTCATCATCGAA | 21 |
| AKT2 | NM_00162 S4727/AKT2.p3 | CAGGTCACGTCCGAGGTCGACACA | 24 |
| APC | NM_00003 S00221APC.f4 | GGACAGCAGGAATGTGTTTC | 20 |
| APC | NM_00003 S0024/APC.r4 | ACCCACTCGATTTGTTTCTG | 20 |
| APC | NM_00003 S4888/APC.p4 | CATTGGCTCCCCGTGACCTGTA | 22 |
| BAD | NM_03298 S2011/BAD.f1 | GGGTCAGGTGCCTCGAGAT | 19 |
| BAD | NM_03298 S2012BAD.r1 | CTGCTCACTCGGCTCAAACTC | 21 |
| BAD | NM_03298 S5058/BAD.p1 | TGGGCCCAGAGCATGTTCCAGATC | 24 |
| BAG1 | NM_00432 S1386/BAG1.f2 | CGTTGTCAGCACTTGGAATACAA | 23 |
| BAG1 | NM_00432 S1387/BAG1.r2 | GTTCAACCTCTTCCTGTGGACTGT | 24 |
| BAG1 | NM_00432 S4731/BAG1.p2 | CCCAATTAACATGACCCGGCAACCAT | 26 |
| BBC3 | NM_01441 S1584/BBC3.f2 | CCTGGAGGGTCCTGTACAAT | 20 |
| BBC3 | NM_01441 S1585/BBC3.r2 | CTAATTGGGCTCCATCTCG | 19 |
| BBC3 | NM_01441 S4890/BBC3.p2 | CATCATGGGACTCCTGCCCTTACC | 24 |
| Bcl2 | NM_00063 S0043/Bcl2.f2 | CAGATGGACCTAGTACCCACTGAGA | 25 |
| Bcl2 | NM_00063 S0045/Bcl2.r2 | CCTATGATTTAAGGGCATTTTTCC | 24 |
| Bcl2 | NM_00063 S4732/Bcl2.p2 | TTCCACGCCGAAGGACAGCGAT | 22 |
| CCND1 | NM_00175 S0058/CCND1.f3 | GCATGTTCGTGGCCTCTAAGA | 21 |
| CCND1 | NM_00175 S0060/CCND1.r3 | CGGTGTAGATGCACAGCTTCTC | 22 |
| CCND1 | NM_00175 S4986/CCND1.p3 | AAGGAGACCATCCCCCTGACGGC | 23 |
| CD18 | NM_00021 S0061/CD18.f2 | CGTCAGGACCCACCATGTCT | 20 |
| CD18 | NM_00021 S0063/CD18.r2 | GGTTAATTGGTGACATCCTCAAGA | 24 |
| CD18 | NM_00021 S4987/CD18.p2 | CGCGGCCGAGACATGGCTTG | 20 |
| CD31 | NM_00044 S1407/CD31.f3 | TGTATTTCAAGACCTCTGTGCACTT | 25 |
| CD31 | NM_00044 S1408/CD31.r3 | TTAGCCTGAGGAATTGCTGTGTT | 23 |
| CD31 | NM_00044 S4939/CD31.p3 | TTTATGAACCTGCCCTGCTCCCACA | 25 |
| CD3z | NM_00073 S0064/CD3z.f1 | AGATGAAGTGGAAGGCGCTT | 20 |
| CD3z | NM_00073 S0066/CD3z.r1 | TGCCTCTGTAATCGGCAACTG | 21 |
| CD3z | NM_00073 S4988/CD3z.p1 | CACCGCGGCCATCCTGCA | 18 |
| CD9 | NM_00176 S0686/CD9.f1 | GGGCGTGGAACAGTTTATCT | 20 |
| CD9 | NM_00176 S06871CD9.r1 | CACGGTGAAGGTTTCGAGT | 19 |
| CD9 | NM_00176 S4792/CD9.p1 | AGACATCTGCCCCAAGAAGGACGT | 24 |
| CDC20 | NM_00125 S4447/CDC20.f1 | TGGATTGGAGTTCTGGGAATG | .21 |
| CDC20 | NM_00125 S4448/CDC20.r1 | GCTTGCACTCCACAGGTACACA | 22 |

(continued)

| | | | |
|---|---|---|---|
| CDC20 | NM_00125 S4449/CDC20.p1 | ACTGGCCGTGGCACTGGACAACA | 23 |
| CDC25B | NM_02187 S1160/CDC25B.f1 | AAACGAGCAGTTTGCCATCAG | 21 |
| CDC25B | NM_02187 S1161/CDC25B.r1 | GTTGGTGATGTTCCGAAGCA | 20 |
| CDC25B | NM_02187 S4842/CDC25B.p1 | CCTCACCGGCATAGACTGGAAGCG | 24 |
| CEGP1 | NM_02097 S1494/CEGP1.f2 | TGACAATCAGCACACCTGCAT | 21 |
| CEGP1 | NM_02097 S1495/CEGP1.r2 | TGTGACTACAGCCGTGATCCTTA | 23 |
| CEGP1 | NM_02097 S4735/CEGP1.p2 | CAGGCCCTCTTCCGAGCGGT | 20 |
| CGA (CHG | NM_00127 S3221/CGA (C.f3 | CTGAAGGAGCTCCAAGACCT | 20 |
| CGA (CHG | NM_00127 S3222/CGA (C.r3 | CAAAACCGCTGTGTTTCTTC | 20 |
| CGA (CHG | NM_00127 S3254/CGA (C.p3 | TGCTGATGTGCCCTCTCCTTGG | 22 |
| COL1A1 | NM_00008 S4531/COL1A1.f1 | GTGGCCATCCAGCTGACC | 18 |
| COL1A1 | NM_00008 S4532/COL1A1.r1 | CAGTGGTAGGTGATGTTCTGGGA | 23 |
| COL1A1 | NM_00008S4533/COL1A1.p1 | TCCTGCGCCTGATGTCCACCG | 21 |
| COL1A2 | NM_00008 S4534/COL1A2.f1 | CAGCCAAGAACTGGTATAGGAGCT | 24 |
| COL1A2 | NM_00008 S4535/COL1A2.r1 | AAACTGGCTGCCAGCATTG | 19 |
| COL1A2 | NM_00008 S4536/COL1A2.p1 | TCTCCTAGCCAGACGTGTTTCTTGTCCTTG | 30 |
| Contig 510 | XM_05894 S2070/Contig.f1 | CGACAGTTGCGATGAAAGTTCTAA | 24 |
| Contig 510 | XM_05894 S2071/Contig.r1 | GGCTGCTAGAGACCATGGACAT | 22 |
| Contig 510 | XM_05894 S5059/Contig.p1 | CCTCCTCCTGTTGCTGCCACTAATGCT | 27 |
| CRABP1 | NM_00437 S5441/CRABP1.f3 | AACTTCAAGGTCGGAGAAGG | 20 |
| CRABP1 | NM_00437 S5442/CRABP1.r3 | TGGCTAAACTCCTGCACTTG | 20 |
| CRABP1 | NM_00437 S5443/CRABP1.p3 | CCGTCCACGGTCTCCTCCTCA | 21 |
| CRIP2 | NM_00131 S5676/CRIP2.f3 | GTGCTACGCCACCCTGTT | 18 |
| CRIP2 | NM_00131 S5677/CRIP2.r3 | CAGGGGCTTCTCGTAGATGT | 20 |
| CRIP2 | NM_00131 S5678/CRIP2.p3 | CCGATGTTCACGCCTTTGGGTC | 22 |
| CYBA | NM_00010 S5300/CYBA.f1 | GGTGCCTACTCCATTGTGG | 19 |
| CYBA | NM_00010 S53011CYBA.r1 | GTGGAGCCCTTCTTCCTCTT | 20 |
| CYBA | NM_00010 S5302/CYBA.p1 | TACTCCAGCAGGCACACAAACACG | 24 |
| DHPS | NM_01340 S4519/DHPS.f3 | GGGAGAACGGGATCAATAGGAT | 22 |
| DHPS | NM_01340 S4520/DHPS.r3 | GCATCAGCCAGTCCTCAAACT | 21 |
| DHPS | NM_01340 S4521/DHPS.p3 | CTCATTGGGCACCAGCAGGTTTCC | 24 |
| DICER1 | NM_17743 S5294/DICER1.f2 | TCCAATTCCAGCATCACTGT | 20 |
| DICER1 | NM_17743 S5295/DICER1.r2 | GGCAGTGAAGGCGATAAAGT | 20 |
| DICER1 | NM_17743 S5296/DICER1.p2 | AGAAAAGCTGTTTGTCTCCCCAGCA | 25 |
| DR4 | NM_00384 S2532/DR4.f2 | TGCACAGAGGGTGTGGGTTAC | 21 |
| DR4 | NM_00384 S2533/DR4.r2 | TCTTCATCTGATTTACAAGCTGTACATG | 28 |
| DR4 | NM_00384 S4981/DR4.p2 | CAATGCTTCCAACAATTTGTTTGCTTGCC | 29 |
| E2F1 | NM_00522 S3063/E2F1.f3 | ACTCCCTCTACCCTTGAGCA | 20 |
| E2F1 | NM_00522 S3064/E2F1.r3 | CAGGCCTCAGTTCCTTCAGT | 20 |
| E2F1 | NM_00522 S4821/E2F1.p3 | CAGAAGAACAGCTCAGGGACCCCT | 24 |
| ER2 | NM_00143 S0109/ER2.f2 | TGGTCCATCGCCAGTTATCA | 20 |
| ER2 | NM_00143 S0111/ER2.r2 | TGTTCTAGCGATCTTGCTTCACA | 23 |
| ER2 | NM_00143 S5001/ER2.p2 | ATCTGTATGCGGAACCTCAAAAGAGTCCCT | 30 |
| ErbB3 | NM_00198 S0112/ErbB3.f1 | CGGTTATGTCATGCCAGATACAC | 23 |
| ErbB3 | NM_00198 S0114/ErbB3.r1 | GAACTGAGACCCACTGAAGAAAGG | 24 |
| ErbB3 | NM_00198 S5002/ErbB3.p1 | CCTCAAAGGTACTCCCTCCTCCCGG | 25 |
| ERBB4 | NM_00523 S1231/ERBB4.f3 | TGGCTCTTAATCAGTTTCGTTACCT | 25 |
| ERBB4 | NM_00523 S1232/ERBB4.r3 | CAAGGCATATCGATCCTCATAAAGT | 25 |
| ERBB4 | NM_00523 S4891/ERBB4.p3 | TGTCCCACGAATAATGCGTAAATTCTCCAG | 30 |
| ERCC1 | NM_00198 S2437/ERCC1.f2 | GTCCAGGTGGATGTGAAAGA | 20 |
| ERCC1 | NM_00198 S2438/ERCC1.r2 | CGGCCAGGATACACATCTTA | 20 |

(continued)

| | | | |
|---|---|---|---|
| ERCC1 | NM_00198 S4920/ERCC1.p2 | CAGCAGGCCCTCAAGGAGCTG | 21 |
| ERK1 | Z11696 S1560/ERK1.f3 | ACGGATCACAGTGGAGGAAG | 20 |
| ERK1 | Z11696 S1561/ERK1.r3 | CTCATCCGTCGGGTCATAGT | 20 |
| ERK1 | Z11696 ; S4882/EkK1.p3 | CGCTGGCTCACCCCTACCTG | 20 |
| ESRRG | NM_00143 S6130/ESRRG.f3 | CCAGCACCATTGTTGAAGAT | 20 |
| ESRRG | NM_00143S6131/ESRRG.r3 | AGTCTCTTGGGCATCGAGTT | 20 |
| ESRRG | NM_00143 S6132/ESRRG.p3 | CCCCAGACCAAGTGTGAATACATGCT | 26 |
| fasl | NM_00063 S0121/fasl.f2 | GCACTTTGGGATTCTTTCCATTAT | 24 |
| fasl | NM_00063 S0123/fasl.r2 | GCATGTAAGAAGACCCTCACTGAA | 24 |
| fasl | NM_00063 S5004/fasl.p2 | ACAACATTCTCGGTGCCTGTAACAAAGAA | 29 |
| FBXO5 | NM_01217 S52017/FBXO5.r1 | GGATTGTAGACTGTCACCGAAATTC | 25 |
| FBXO5 | NM_01217 S2018/FBXO5.f1 | GGCTATTCCTCATTTTCTCTACAAAGTG | 28 |
| FBXO5 | NM_01217 S5061/FBXO5.p1 | CCTCCAGGAGGCTACCTTCTTCATGTTCiAC | 30 |
| FHIT | NM_00201 S2443/FHIT.f1 | CCAGTGGAGCGCTTCCAT | 18 |
| FHIT | NM_00201 S2444/FHIT.r1 | CTCTCTGGGTCGTCTGAAACAA | 22 |
| FHIT | NM_00201 S4921/FHIT.p1 | TCGGCCACTTCATCAGGACGCAG | 23 |
| FUS | NM_00496 S2936/FUS.f1 | GGATAATTCAGACAACAACACCATCT | 26 |
| FUS | NM_00496 S2937/FUS.r1 | TGAAGTAATCAGCCACAGACTCAAT | 25 |
| FUS | NM_00496 S4801/FUS.p1 | TCAATTGTAACATTCTCACCCAGGCCTTG | 29 |
| FYN | NM_00203 S5695/FYN.f3 | GAAGCGCAGATCATGAAGAA | 20 |
| FYN | NM_00203 S5696/FYN.r3 | CTCCTCAGACACCACTGCAT | 20 |
| FYN | NM_00203 S5697/FYN.p3 | CTGAAGCACGACAAGCTGGTCCAG | 24 |
| G-Catenin | NM_00223 S2153/G.Cate.f1 | TCAGCAGCAAGGGCATCAT | 19 |
| G-Catenin | NM_00223 S2154/G-Cate.r1 | GGTGGTTTTCTTGAGCGTGTACT | 23 |
| G-Catenin | NM_00223 S5044/G-Cate.p1 | CGCCCGCAGGCCTCATCCT | 19 |
| GATA3 | NM_00205 S0127/GATA3.f3 | CAAAGGAGCTCACTGTGGTGTCT | 23 |
| GATA3 | NM_00205 S0129/GATA3.r3 | GAGTCAGAATGGCTTATTCACAGATG | 26 |
| GATA3 | NM 00205 S5005/GATA3.p3 | TGTTCCAACCACTGAATCTGGACC | 24 |
| GBP1 | NM_00205 S5698/GBP1.f1 | TTGGGAAATATTTGGGCATT | 20 |
| GBP1 | NM_00205 S5899/GBP1.r1 | AGAAGCTAGGGTGGTTGTCC | 20 |
| GBP1 | NM_00205 S5700/GBP1.p1 | TTGGGACATTGTAGACTTGGCCAGAC | 26 |
| GBP2 | NM_00412 S5707/GBP2.f2 | GCATGGGAACCATCAACCA | 19 |
| GBP2 | NM_00412 S5708/GBP2.r2 | TGAGGAGTTTGCCTTGATTCG | 21 |
| GBP2 | NM_00412 S5709/GBP2.p2 | CCATGGACCAACTTCACTATGTGACAGAGC | 30 |
| GGPS1 | NM_00483 S1590/GGPS1.f1 | CTCCGACGTGGCTTTCCA | 18 |
| GGPS1 | NM_00483 S1591/GGPS1.r1 | CGTAATTGGCAGAATTGATGACA | 23 |
| GGPS1 | NM_00483 S4896/GGPS1.p1 | TGGCCCACAGCATCTATGGAATCCC | 25 |
| GRB7 | NM_00531 S0130/GRB7.f2 | CCATCTGCATCCATCTTGTT | 20 |
| GRB7 | NM_00531 S0132/GRB7.r2 | GGCCACCAGGGTATTATCTG | 20 |
| GRB7 | NM_00531 S4726/GRB7.p2 | CTCCCCACCCTTGAGAAGTGCCT | 23 |
| Hepsin | NM_00215 S2269/Hepsin.f1 | AGGCTGCTGGAGGTCATCTC | 20 |
| Hepsin | NM_00215 S2270/Hepsin.r1 | CTTCCTGCGGCCACAGTCT | 19 |
| Hepsin | NM_00215 S2271/Hepsin.p1 | CCAGAGGCCGTTTCTTGGCCG | 21 |
| HLA-DPB1 | NM_00212 S4573/HLA-DP.f1 | TCCATGATGGTTCTGCAGGTT | 21 |
| HLA-DPB1 | NM_00212 S4574/HLA-DP.r1 | TGAGCAGCACCATCAGTAACG | 21 |
| HLA-DPB1 | NM_00212 S4575/HLA-DP.p1 | CCCCGGACAGTGGCTCTGACG | 21 |
| ID2 | NM_00216 S0151/ID2.f4 | AACGACTGCTACTCCAAGCTCAA | 23 |
| ID2 | NM_00216 S0153/ID2.r4 | GGATTTCCATCTTGCTCACCTT | 22 |
| ID2 | NM_00216 S5009/ID2.p4 | TGCCCAGCATCCCCCAGAACAA | 22 |
| IGF1R | NM_00087 S1249/IGF1R.f3 | GCATGGTAGCCGAAGATTTCA | 21 |
| IGF1R | NM_00087 S1250/IGF1R.r3 | TTTCCGGTAATAGTCTGTCTCATAGATATC | 30 |

(continued)

| | | | |
|---|---|---|---|
| IGF1R | NM_00087 S4895/IGF1R.p3 | CGCGTCATACCAAAATCTCCGATTTTGA | 28 |
| IL6 | NM_00060 S0760/IL6.f3 | CCTGAACCTTCCAAAGATGG | 20 |
| IL6 | NM_00060 S0761/IL6.r3 | ACCAGGCAAGTCTCCTCATT | 20 |
| IL6 | NM_00060 S4800/IL6.p3 | CCAGATTGGAAGCATCCATCTTTTTCA | 27 |
| ILT-2 | NM_00666 S1811/ILT-2.f2 | AGCCATCACTCTCAGTGCAG | 20 |
| ILT-2 | NM_00666S1612/ILT-2.r2 | ACTGCAGAGTCAGGGTCTCC | 20 |
| ILT-2 | NM_00666 S4904/ILT-2.p2 | CAGGTCCTATCGTGGCCCCTGA | 22 |
| IRS1 | NM_00554 S1943/IRS1.f3 | CCACAGCTCACCTTCTGTCA | 20 |
| IRS1 | NM_00554 S1944/IRS1.r3 | CCTCAGTGCCAGTCTCTTCC | 20 |
| IRIS1 | NM_00554 S5050/IRS1.p3 | TCCATCCCAGCTCCAGCCAG | 20 |
| KRT18 | NM_00022 S17101KRT18.f2 | AGAGATCGAGGCTCTCAAGG | 20 |
| KRT18 | NM_00022 S1711/KRT18.r2 | GGCCTTTTACTTCCTCTTCG | 20 |
| KRT18 | NM_00022 S4762/KRT18.p2 | TGGTTCTTCTTCATGAAGAGCAGCTCC | 27 |
| MAPK14 | NM_13901 S5557/MAPK14.f2 | TGAGTGGAAAAGCCTGACCTATG | 23 |
| MAPK14 | NM_13901 S5558/MAPK14.r2 | GGACTCCATCTCTTCTTGGTCAA | 23 |
| MAPK14 | NM_13901 S5559/MAPK14.p2 | TGAAGTCATCAGCTTTGTGCCACCACC | 27 |
| MCM2 | NM_00452 S1602/MCM2.f2 | GACTTTTGCCCGCTACCTTTC | 21 |
| MCM2 | NM_00452 S1603/MCM2.r2 | GCCACTAACTGCTTCAGTATGAAGAG | 26 |
| MCM2 | NM_00452 S4900/MCM2.p2 | ACAGCTCATTGTTGTCACGCCGGA | 24 |
| MCM6 | NM_00591 S1704/MCM6.f3 | TGATGGTCCTATGTGTCACATTCA | 24 |
| MCM6 | NM_00591 S1705/MCM6.r3 | TGGGACAGGAAACACACCAA | 20 |
| MCM6 | NM_00591 S4919/MCM6.p3 | CAGGTTTCATACCAACACAGGCTTCAGCAC | 30 |
| MCP1 | NM_00298 S1955/MCP1.f1 | CGCTCAGCCAGATGCAATC | 19 |
| MCP1 | NM_00298 S1956/MCP1.r1 | GCACTGAGATCTTCCTATTGGTGAA | 25 |
| MCP1 | NM_00298 S5052/MCP1.p1 | TGCCCCAGTCACCTGCTGTTA | 21 |
| MGMT | NM_00241 S1922/MGMT.f1 | GTGAAATGAAACGCACCACA | 20 |
| MGMT | NM_00241 S1923/MGMT.r1 | GACCCTGCTCACAACCAGAC | 20 |
| MGMT | NM_00241 S5045/MGMT.p1 | CAGCCCTTTGGGGAAGCTGG | 20 |
| MMP12 | NM_00242 S4381/MMP12.f2 | CCAACGCTTGCCAAATCCT | 19 |
| MMP12 | NM_00242 S4382/MMP12.r2 | ACGGTAGTGACAGCATCAAAACTC | 24 |
| MMP12 | NM_00242 S4383/MMP12.p2 | AACCAGCTCTCTGTGACCCCAATT | 24 |
| MSH3 | NM_00243 S5940/MSH3.f2 | TGATTACCATCATGGCTCAGA | 21 |
| MSH3 | NM_00243 S5941/MSH3.r2 | CTTGTGAAAATGCCATCCAC | 20 |
| MSH3 | NM_00243 S5942/MSH3.p2 | TCCCAATTGTCGCTTCTTCTGCAG | 24 |
| MTA1 | NM_00468 S2369/MTA1.f1 | CCGCCCTCACCTGAAGAGA | 19 |
| MTA1 | NM_00468 S2370/MTA1.r1 | GGAATAAGTTAGCCGCGCTTCT | 22 |
| MTA1 | NM_00468 S4855/MTA1.p1 | CCCAGTGTCCGCCAAGGAGCG | 21 |
| MUC1 | NM_00245 S0782/MUC1.f2 | GGCCAGGATCTGTGGTGGTA | 20 |
| MUC1 | NM_00245 S0783/MUC1.r2 | CTCCACGTCGTGGACATTGA | 20 |
| MUC1 | NM_00245 S4807/MUC1.p2 | CTCTGGCCTTCCGAGAAGGTACC | 23 |
| NPD009 | (ANM_02068 S4474/NPD009.f3 | GGCTGTGGCTGAGGCTGTAG | 20 |
| NPD009 | (ANM_02068 S4475/NPD009.r3 | GGAGCATTCGAGGTCAAATCA | 21 |
| NPD009 | (ANM_02068 S4476/NPD009.p3. | TTCCCAGAGTGTCTCACCTCCAGCAGAG | 28 |
| PR | NM_00092S1336/PR.f6 | GCATCAGGCTGTCATTATGG | 20 |
| PR | NM_00092 S1337/PR.r6 | AGTAGTTGTGCTGCCCTTCC | 20 |
| PR | NM_00092 S4743/PR.p6 | TGTCCTTACCTGTGGGAGCTGTAAGGTC | 28 |
| PRKCD | NM_00625 S1738/PRKCD.f2 | CTGACACTTGCCGCAGAGAA | 20 |
| PRKCD | NM_00625 S1739/PRKCD.r2 | AGGTGGTCCTTGGTCTGGAA | 20 |
| PRKCD | NM_00625 S4923/PRKCD.p2 | CCCTTTCTCACCCACCTCATCTGCAC | 26 |
| PTPD1 | NM_00703 S3069/PTPD1.f2 | CGCTTGCCTAACTCATACTTTCC | 23 |
| PTPD1 | NM_00703 S3070/PTPD1.r2 | CCATTCAGACTGCGCCACTT | 20 |

(continued)

| | | | |
|---|---|---|---|
| PTPD1 | NM_00703 S4822/PTPD1.p2 | TCCACGCAGCGTGGCACTG | 19 |
| RAB6C | NM_03214 S5535/RAB6C.f1 | GCGACAGCTCCTCTAGTTCCA | 21 |
| RAB6C | NM_03214 S5537/RAB6C.p1 | TTCCCGAAGTCTCCGCCCG | 19 |
| RAB6C | NM_03214 S5538/RAB6C.r1 | GGAACACCAGCTTGAATTTCCT | 22 |
| RALBP1 | NM_00678 S5853/RALBP1.f1 | GGTGTCAGATATAAATGTGCAAATGC | 26 |
| RALBP1 | NM_0067855854/RALBP1.r1 | TTCGATATTGCCAGCAGCTATAAA | 24 |
| RALBP1 | NM_00678 S5855/RALBP1.p1 | TGCTGTCCTGTCGGTCTCAGTACGTTCA | 28 |
| RAP1GDS | NM_02115S5306/RAP1GD.f2 | TGTGGATGCTGGATTGATTT | 20 |
| RAP1GDS | NM_02115 S5307/RAP1GD.r2 | AAGCAGCACTTCCTGGTCTT | 20 |
| RAP1GDS | NM_02115 S5308/RAP1GD.p2 | CCACTGGTGCAGCTGCTAAATAGCA | 25 |
| RASSF1 | NM_00718 S2393/RASSF1.f3 | AGTGGGAGACACCTGACCTT | 20 |
| RASSF1 | NM_00718 S2394/RASSF1.r3 | TGATCTGGGCATTGTACTCC | 20 |
| RASSF1 | NM_00718 S4909/RASSF1.p3 | TTGATCTTCTGCTCAATCTCAGCTTGAGA | 29 |
| rhoC | NM_00516 S2162/rhoC.f1 | CCCGTTCGGTCTGAGGAA | 18 |
| rhoC | NM_00516 S2163/rhoC.r1 | GAGCACTCAAGGTAGCCAAAGG | 22 |
| rhoC | NM_00516 S5042/rhoC.p1 | TCCGGTTCGCCATGTCCCG | 19 |
| RUNX1 | NM_00175 S4588/RUNX1.f2 | AACAGAGACATTGCCAACCA | 20 |
| RUNX1 | NM_00175 S4589/RUNX1.r2 | GTGATTTGCCCAGGAAGTTT | 20 |
| RUNX1 | NM_00175 S4590/RUNX1.p2 | TTGGATCTGCTTGCTGTCCAAACC | 24 |
| SEMA3F | NM_00418 S2857/SEMA3F.f3 | CGCGAGCCCCTCATTATACA | 20 |
| SEMA3F | NM_00418 S2858/SEMA3F.r3 | CACTCGCCGTTGACATCCT | 19 |
| SEMA3F | NM_00418 S4972/SEMA3F.p3 | CTCCCCACAGCGCATCGAGGAA | 22 |
| SGCB | NM_00023 S5752/SGCB.f1 | CAGTGGAGACCAGTTGGGTAGTG | 23 |
| SGCB | NM_00023 S5T53/SGCB.r1 | CCTTGAAGAGCGTCCCATCA | 20 |
| SGCB | NM_00023 S5754/SGCB.p1 | CACACATGCAGAGCTTGTAGCGTACCCA | 28 |
| STAT1 | NM_00731 S1542/STAT1.f3 | GGGCTCAGCTTTCAGAAGTG | 20 |
| STAT1 | NM_00731 S1543/STAT1.r3 | ACATGTTCAGCTGGTCCACA | 20 |
| STAT1 | NM_00731 S4878/STAT1.p3 | TGGCAGTTTTCTTCTGTCACCAAAA | 25 |
| STAT3 | NM_00315 S1545/STAT3.f1 | TCACATGCCACTTTGGTGTT | 20 |
| STAT3 | NM_00315 S1546/STAT3.r1 | CTTGCAGGAAGCGGCTATAC | 20 |
| STAT3 | NM_00315 S4881/STAT3.p1 | TCCTGGGAGAGATTGACCAGCA | 22 |
| TBP | NM_00319 S0262/TBP.f1 | GCCCGAAACGCCGAATATA | 19 |
| TBP | NM_00319 S0264/TBP.r1 | CGTGGCTCTCTTATCCTCATGAT | 23 |
| TBP | NM 00319 S4751/TBP.p1 | TACCGCAGCAAACCGCTTGGG | 21 |
| TK1 | NM_00325 S0866/TK1.f2 | GCCGGGAAGACCGTAATTGT | 20 |
| TK1 | NM_00325 S0927/TK1.r2 | CAGCGGCACCAGGTTCAG | 18 . |
| TK1 | NM_00325 S4798/TK1.p2 | CAAATGGCTTCCTCTGGAAGGTCCCA | 26 |
| TP53BP1 | NM_00565 S1747/TP53BP.f2 | TGCTGTTGCTGAGTCTGTTG | 20 |
| TP53BP1 | NM_00565 S1748/TP53BP.r2 | CTTGCCTGGCTTCACAGATA | 20 |
| TP53BP1 | NM_00565 S4924/TP53BP.p2 | CCAGTCCCCAGAAGACCATGTCTG | 24 |
| TUBB | NM 00106 S826/TUBB.f3 | TGTGGTGAGGAAGGAGTCAG | 20 |
| TUBB | NM_00106 S5827/TUBB.r3 | CCCAGAGAGTGGGTCAGC | 18 |
| TUBB | NM_00106 S5828/TUBB.p3 | CTGTGACTGTCTCCAGGGCTTCCA | 24 |
| VCAM1 | NM_00107 S3505/VCAM1.f1 | TGGCTTCAGGAGCTGAATACC | 21 |
| VCAM1 | NM_00107 S3506/VCAM1.r1 | TGCTGTCGTGATGAGAAAATAGTG | 24 |
| VCAM1 | NM_00107 S3507/VCAM1.p1 | CAGGCACACACAGGTGGGACACAAAT | 26 |
| Wnt-5a | NM_00339 S6183/Wnt-5a.f1 | GTATCAGGACCACATGCAGTACATC | 25 |
| Wnt-5a | NM_00339 S6184/Wnt-5a.r1 | TGTCGGAATTGATACTGGCATT | 22 |
| Wnt.5a | NM_00339 S6185/Wnt-5a.p1 | TTGATGCCTGTCTTCGCGCCTTCT | 24 |
| ZNF38 | NM_14591 S5593/ZNF38.f3 | TTTCCAAACATCAGCGAGTC | 20 |
| ZNF38 | NM_14591 S5594/ZNF38.r3 | AACAGGAGCGCTTGAAAGTT | 20 |

(continued)

| | | | |
|---|---|---|---|
| ZNF38 | NM_14591 S5595/ZNF38.p3 | ACGGTGCTTCTCCCTCTCCAGTG | 23 |

**TABLE 3**

| | | Sequence |
|---|---|---|
| A-Catenin | NM_00190 | CGTTCCGATCCTCTATACTGCATCCCAGGCATG CCTACAGCACCCTGATGTCGCAGCCTATAAG-GCCAACAGGGACCT |
| ABCC5 | NM_00568 | TGCAGACTGTACCATGCTGACCATTGCCCATCG CCTGCACACGGTTCTAGGCTCCGATAGGAT-TATGGTGCTGGCC |
| AK055699 | AK055699 | CTGCATGTGATTGAATAAGAAACAAGAAAGTGA CCACACCAAAGCCTCCCTGGCTGGTGTACAG-GGATCAGGTCCACA |
| AKAP-2 | NM_00720 | ACGAATTGTCGGTGAGGTCTCAGGATACCACAG TCCTGGAGACCCTATCCAATGATTTCAGCAT-GGAC |
| AKT1 | NM_00516 | CGCTTCTATGGCGCTGAGATTGTGTCAGCCCTG GACTACCTGCACTCGGAGAAGAACGTGGTG-TACCGGGA |
| AKT2 | NM_00162 | TCCTGCCACCCTTCAAACCTCAGGTCACGTCCG AGGTCGACACAAGGTACTTCGATGATGAATT-TACCGCC |
| APC | NM_00003 | GGACAGCAGGAATGTGTTTCTCCATACAGGTCA CGGGGAGCCAATGGTTCAGAAACAAATCGAGT-GGGT |
| BAD | NM_03298 | GGGTCAGGTGCCTCGAGATCGGGCTTGGGCCC AGAGCATGTTCCAGATCCCAGAGTTT-GAGCCGAGTGAGCAG |
| BAG1 | NM_00432 | CGTTGTCAGCACTTGGAATACAAGATGGTTGCC GGGTCATGTTAATTGGGAAAAAGAACAGTC-CACAGGAAGAGGTTGAAC |
| BBC3 | NM_01441 | CCTGGAGGGTCCTGTACAATCTCATCATGGGAC TCCTGCCCTTACCCAGGGGCCACA-GAGCCCCCGAGATGGAGCCCAATTAG |
| Bcl2 | NM_00063 | CAGATGGACCTAGTACCCACTGAGATTTCCACG CCGAAGGACAGCGATGGGAAAAATGCCCT-TAAATCATAGG |
| CCND1 | NM_00175 | GCATGTTCGTGGCCTCTAAGATGAAGGAGACCA TCCCCCTGACGGCCGAGAAGCTGTGCATCTA-CACCG |

(continued)

| | | Sequence |
|---|---|---|
| CD18 | NM_00021 | CGTCAGGACCCACCATGTCTGCCCCATCACGC GGCCGAGACATGGCTTGGCCACAGCTCTTGAG-GATGTCACCAATTAACC |
| CD31 | NM_00044 | TGTATTTCAAGACCTCTGTGCACTTATTTATGAA CCTGCCCTGCTCCCACAGAACACAGCAATTC-CTCAGGCTAA |
| CD3z | NM_00073 | AGATGAAGTGGAAGGCGCTTTTCACCGCGGCC ATCCTGCAGGCACAGTTGCCGATTACAGAGGCA |
| CD9 | NM_00176 | GGGCGTGGAACAGTTTATCTCAGACATCTGCCC CAAGAAGGACGTACTCGAAAGCTTCACCGTG |
| CDC20 | NM_00125 | TGGATTGGAGTTCTGGGAATGTACTGGCCGTG GCACTGGACAACAGTGTGTACCTGTGGAGT-GCAAGC |
| CDC25B | NM_02187 | AAACGAGCAGTTTGCCATCAGACGCTTCCAGTC TATGCCGGTGAGGCTGCT-GGGCCACAGCCCCGTGCTTCGGAACATCAC-CAAC |
| CEGP1 | NM_02097 | TGACAATCAGCACACCTGCATTCACCGCTCGGA AGAGGGCCTGAGCTGCATGAATAAGGATCACG-GCTGTAGTCACA |
| CGA (CHG | NM_00127 | CTGAAGGAGCTCCAAGACCTCGCTCTCCAAGG CGCCAAGGAGAGGGCACATCAGCAGAA-GAAACACAGCGGTTTTG |
| COL1A1 | NM_00008 | GTGGCCATCCAGCTGACCTTCCTGCGCCTGAT GTCCACCGAGGCCTCCCAGAACATCACCTAC-CACTG |
| COL1A2 | NM_00008 | CAGCCAAGAACTGGTATAGGAGCTCCAAGGAC AAGAAACACGTCTGGCTAGGAGAAACTATCAAT-GCTGGCAGCCAGTTT |
| Config 510 | XM_05894 | CGACAGTTGCGATGAAA6TTCTAATCTCTTCCCT CCTCCTGTTGCTGCCACTAATGCTGATGTCCAT-GGTCTCTAGCAGCC |

(continued)

| | | Sequence |
|---|---|---|
| CRABP1 | NM_00437 | AACTTCAAGGTCGGAGAAGGCTTTGAGGAGGA GACCGTGGACGGACGCAAGTGCAGGAGTT-TAGCCA |
| CRIP2 | NM_00131 | GTGCTACGCCACCCTGTTCGGACCCAAAGGCG TGAACATCGGGGGCGCGGGCTCCTACATCTAC-GAGAAGCCCCTG |
| CYBA | NM_00010 | GGTGCCTACTCCATTGTGGCGGGCGTGTTTGT GTGCCTGCTGGAGTACCCCCGGGGGAAGAG-GAAGAAGGGCTCCAC |
| DHPS | NM_01340 | GGGAGAACGGGATCAATAGGATCGGAAACCTG CTGGTGCCCAATGAGAATTACTGCAAGTTTGAG-GACTGGCTGATGC |
| DICER1 | NM_17743 | TCCAATTCCAGCATCACTGTGGAGAAAAGCTGT TTGTCTCCCCAGCATACTTTATCGCCTTCACT-GCC |
| DR4 | NM_00384 | TGCACAGAGGGTGTGGGTTACACCAATGCTTCC AACAATTTGTTTGCTTGCCTCCCATGTA-CAGCTTGTAAATCAGATGAAGA |
| E2F1 | NM_00522 | ACTCCCTCTACCCTTGAGCAAGGGCAGGGGTC CCTGAGCTGTTCTTCTGCCCCATACTGAAG-GAACTGAGGCCTG |
| ER2 | NM_00143 | TGGTCCATCGCCAGTTATCACATCTGTATGCGG AACCTCAAAAGAGTCCCTGGTGTGAAGCAA-GATCGCTAGAACA |
| ErbB3 | NM_00198 | CGGTTATGTCATGCCAGATACACACCTCAAAGG TACTCCCTCCTCCCGGGAAGGCACCCTTTCT-TCAGTGGGTCTCAGTTC |
| ERBB4 | NM_00523 | TGGCTCTTAATCAGTTTCGTTACCTGCCTCTGG AGAATTTACGCATTATTCGTGGGACAAAACTT-TATGAGGATCGATATGCCTTG |
| ERCC1 | NM_00198 | GTCCAGGTGGATGTGAAAGATCCCCAGCAGGC CCTCAAGGAGCTGGCTAAGATGTGTATCCT-GGCCG |

(continued)

| | | Sequence |
|---|---|---|
| ERK1 | Z11696 | ACGGATCACAGTGGAGGAAGCGCTGGCTCACC CCTACCTGGAGCAGTACTATGACCCGACGGAT-GAG |
| ESRRG | NM_00143 | CCAGCACCATTGTTGAAGATCCCCAGACCAAGT GTGAATACATGCTCAACTCGATGCCCAAGA-GACT |
| fasl | NM_00063 | GCACTTTGGGATTCTTTCCATTATGATTCTTTGT TACAGGCACCGAGAATGTTGTATTCAGTGAG-GGTCTTCTTACATGC |
| FBXO5 | NM_01217 | GGCTATTCCTCATTTTCTCTACAAAGTGGCCTCA GTGAACATGAAGAAGGTAGCCTCCTGGAGGA-GAATTTCGGTGACAGTCTACAATCC |
| FHIT | NM_00201 | CCAGTGGAGCGCTTCCATGACCTGCGTCCTGAT GAAGTGGCCGATTTGTTTCAGACGACCCAGA-GAG |
| FUS | NM_00496 | GGATAATTCAGACAACAACACCATCTTTGTGCA AGGCCTGGGTGAGAATGTTACAATTGAGTCT-GTGGCTGATTACTTCA |
| FYN | NM_00203 | GAAGCGCAGATCATGAAGAAGCTGAAGCACGA CAAGCTGGTCCAGCTCTATGCAGTGGTGTCT-GAGGAG |
| G-Catenin | MM_00223 | TCAGCAGCAAGGGCATCATGGAGGAGGATGAG GCCTGCGGGCGCCAGTACACGCTCAAGAAAAC-CACC |
| GATA3 | NM_00205 | CAAAGGAGCTCACTGTGGTGTCTGTGTTCCAAC CACTGAATCTGGACCCCATCTGT-GAATAAGCCATTCTGACTC |
| GBP1 | NM_00205 | TTGGGAAATATTTGGGCATTGGTCTGGCCAAGT CTACAATGTCCCAATATCAAGGACAACCAC-CCTAGCTTCT |
| GBP2 | NM_00412 | GCATGGGAACCATCAACCAGCAGGCCATGGAC CAACTTCACTATGTGACAGAGCTGACAGATC-GAATCAAGGCAAACTCCTCA |

(continued)

| | | Sequence |
|---|---|---|
| GGPS1 | NM_00483 | CTCCGACGTGGCTTTCCAGTGGCCCACAGCAT CTATGGAATCCCATCTGTCATCAATTCT-GCCAATTACG |
| GRB7 | NM_00531 | CCATCTGCATCCATCTTGTTTGGGCTCCCCACC CTTGAGAAGTGCCTCAGATAATACCCTGGT-GGCC |
| Hepsin | NM_00215 | AGGCTGCTGGAGGTCATCTCCGTGTGTGATTGC CCCAGAGGCCGTTTCTTGGCCGCCATCT-GCCAAGACTGTGGCCGCAGGAAG |
| HLA-DPB1 | NM_00212 | TCCATGATGGTTCTGCAGGTTTCTGCGGCCCCC CGGACAGTGGCTCTGACGGCGTTACTGATGGT-GCTGCTCA |
| ID2 | NM_00216 | AACGACTGCTACTCCAAGCTCAAGGAGCTGGTG CCCAGCATCCCCCAGAACAAGAAGGTGAGCAA-GATGGAAATCC |
| IGF1R | NM_00087 | GCATGGTAGCCGAAGATTTCACAGTCAAAATCG GAGATTTTGGTATGACGCGAGATATCTATGA-GACAGACTATTACCGGAAA |
| IL6 | NM_00060 | CCTGAACCTTCCAAAGATGGCTGAAAAGATGGA TGCTTCCAATCTGGATTCAATGAGGAGACTT-GCCTGGT |
| ILT-2 | MM_00666 | AGCCATCACTCTCAGTGCAGCCAGGTCCTATCG TGGCCCCTGAGGAGACCCTGACTCTGCAGT |
| IRS1 | NM_00554 | CCACAGCTCACCTTCTGTCAGGTGTCCATCCCA GCTCCAGCCAGCTCCCAGAGAGGAAGAGACT-GGCACTGAGG |
| KRT18 | NM_00022 | AGAGATCGAGGCTCTCAAGGAGGAGCTGCTCT TCATGAAGAAGAACCACGAAGAGGAAG-TAAAAGGCC |
| MAPK14 | NM_13901 | TGAGTGGAAAAGCCTGACCTATGATGAAGTCAT CAGCTTTGTGCCACCACCCCTTGACCAAGAA-GAGATGGAGTCC |

(continued)

| | | Sequence |
|---|---|---|
| MCM2 | NM_00452 | GACTTTTGCCCGCTACCTTTCATTCCGGCGTGA CAACAATGAGCTGTTGCTCTTCATACTGAAG-CAGTTAGTGGC |
| MCM6 | NM_00591 | TGATGGTCCTATGTGTCACATTCATCACAGGTTT CATACCAACACAGGCTTCAGCACTTCCTTTGGT-GTGTTTCCTGTCCCA |
| MCP1 | NM_00298 | CGCTCAGCCAGATGCAATCAATGCCCCAGTCAC CTGCTGTTATAACTTCACCAATAGGAAGATCT-CAGTOC |
| MGMT | NM_00241 | GTGAAATGAAACGCACCACACTGGACAGCCCTT TGGGGAAGCTGGAGCTGTCTGGTTGTGAGCAG-GGTC |
| MMP12 | NM_00242 | CCAACGCTTGCCAAATCCTGACAATTCAGAACC AGCTCTCTGTGACCCCAATTTGAGTTTTGAT-GCTGTCACTACCGT |
| MSH3 | NM_00243 | TGATTACCATCATGGCTCAGATTGGCTCCTATG TTCCTGCAGAAGAAGCGACAATTGGGATTGT-GGATGGCATTTTCACAAG |
| MTA1 | NM_00468 | CCGCCCTCACCTGAAGAGAAACGCGCTCCTTG GCGGACACTGGGGGAGGAGAGGAA-GAAGCGCGGCTAACTTATTCC |
| MUC1 | NM_00245 | GGCCAGGATCTGTGGTGGTACAATTGACTCTGG CCTTCCGAGAAGGTACCATCAATGTCCAC-GACGTGGAG |
| NPD009 | (ANM_02068 | GGCTGTGGCTGAGGCTGTAGCATCTCTGCTGG AGGTGAGACACTCTGGGAACTGATTTGACCTC-GAATGCTCC |
| PR | NM_00092 | GCATCAGGCTGTCATTATGGTGTCCTTACCTGT GGGAGCTGTAAGGTCTTCTTTAAGAGGGCAAT-GGAAGGGCAGCACAACTACT |
| PRKCD | NM_00625 | CTGACACTTGCCGCAGAGAATCCCTTTCTCACC CACCTCATCTGCACCTTCCAGACCAAGGAC-CACCT |

(continued)

| | | Sequence |
|---|---|---|
| PTPD1 | NM_00703 | CGCTTGCCTAACTCATACTTTCCCGTTGACACTT GATCCACGCAGCGTGGCACTGGGACGTAAGT-GGCGCAGTCTGAATGG |
| RAB6C | NM_03214 | GCGACAGCTCCTCTAGTTCCACCATGTCCGCG GGCGGAGACTTCGGGAATCCGCTGAGGAAAT-TCAAGCTGGTGTTCC |
| RALBP1 | NM_00678 | GGTGTCAGATATAAATGTGCAAATGCCTTCTTG CTGTCCTGTCGGTCTCAGTACGTTCACTTTAT-AGCTGCTGGCAATATCGAA |
| RAP1GDS | NM_02115 | TGTGGATGCTGGATTGATTTCACCACTGGTGCA GCTGCTAAATAGCAAAGACCAGGAAGTGCT-GCTT |
| RASSF1 | NM_00718 | AGTGGGAGACACCTGACCTTTCTCAAGCTGAGA TTGAGCAGAAGATCAAGGAGTACAATGCCCA-GATCA |
| rhoC | NM_00516 | CCCGTTCGGTCTGAGGAAGGCCGGGACATGGC GAACCGGATCAGTGCCTTTGGCTACCTTGAGT-GCTC |
| RUNX1. | NM_00175 | AACAGAGACATTGCCAACCATATTGGATCTGCT TGCTGTCCAAACCAGCAAACTTCCTGGGCAAAT-CAC |
| SEMA3F | NM_00418 | CGCGAGCCCCTCATTATACACTGGGCAGCCTC CCCACAGCGCATCGAGGAATGCGTGCTCTCAG-GCAAGGATGTCAACGGCGAGTG |
| SGCB | NM_00023 | CAGTGGAGACCAGTTGGGTAGTGGTGACTGGG TACGCTACAAGCTCTGCATGTGTGCTGAT-GGGACGCTCTTCAAGG |
| STAT1 | NM_00731 | GGGCTCAGCTTTCAGAAGTGCTGAGTTGGCAGT TTTCTTCTGTCACCAAAAGAGGTCTCAATGT-GGACCAGCTGAACATGT |
| STAT3 | NM_00315 | TCACATGCCACTTTGGTGTTTCATAATCTCCTGG GAGAGATTGACCAGCAGTATAGCCGCTTCCT-GCAAG |

(continued)

| | | Sequence |
|---|---|---|
| TBP | NM_00319 | GCCCGAAACGCCGAATATAATCCCAAGCGGTTT GCTGCGGTAATCATGAGGATAAGAGAGCCACG |
| TK1 | NM_00325 | GCCGGGAAGACCGTAATTGTGGCTGCACTGGA TGGGACCTTCCAGAGGAAGCCATTT-GGGGCCATCCTGAACCTGGTGCCGCTG |
| TP53BP1 | NM_00565 | TGCTGTTGCTGAGTCTGTTGCCAGTCCCCAGAA GACCATGTCTGTGTTGAGCTGTATCTGT-GAAGCCAGGCAAG |
| TUBB | NM_00106 | TGTGGTGAGGAAGGAGTCAGAGAGCTGTGACT GTCTCCAGGGCTTCCAGCTGACCCACTCTCT-GGG |
| VCAM1 | NM_00107 | TGGCTTCAGGAGCTGAATACCCTCCCAGGCACA CACAGGTGGGACACAAATAAGGGTTTTGGAAC-CACTATTTTCTCATCACGACAGCA |
| Wnt-5a | NM_00339 | GTATCAGGACCACATGCAGTACATCGGAGAAG GCGCGAAGACAGGCATCAAAGAATGCCAGTAT-CAATTCCGACA |
| ZNF38 | NM_14591 | TTTCCAAACATCAGCGAGTCCACACTGGAGAGG GAGAAGCACCGTAACTTTCAAGCGCTCCTGTT |

## Claims

1. A method for determining whether a level of an RNA transcript, is associated with a beneficial response to chemotherapy treatment comprising an anthracycline and a taxane, the method comprising:

   determining a level of a RNA transcript in a biological sample comprising breast cancer cells obtained before chemotherapy from a human subject diagnosed with breast cancer, wherein the RNA transcript is the RNA transcript of BAG1;
   normalizing the level of the RNA transcript of BAG1 against a level of at least one reference RNA to obtain a normalized expression level of BAG1;
   comparing the normalized expression level of BAG1 from the subject with a normalized expression level of BAG1 in reference breast cancer patient samples obtained before chemotherapy from breast cancer patients treated with chemotherapy comprising an anthracycline and a taxane; and
   determining that the normalized expression level of BAG1 from the subject is associated with a decreased likelihood of a beneficial response to chemotherapy treatment comprising an anthracycline and a taxane if the normalized expression level of BAG1 from the subject is increased compared to the normalized expression level of BAG1 in the reference breast cancer patient samples obtained from patients who exhibited a beneficial response to chemotherapy treatment comprising an anthracycline and a taxane.

2. A method as claimed in claim 1, wherein said chemotherapy is adjuvant chemotherapy or neoadjuvant chemotherapy.

3. A method as claimed in claim 1, wherein said taxane is docetaxel or paclitaxel.

4. A method as claimed in claim 1, wherein said anthracycline is doxorubicin.

5. A method as claimed in claim 1, wherein said breast cancer is an invasive breast cancer.

6. A method as claimed in claim 1, wherein said breast cancer is a stage II or stage III breast cancer.

7. A method as claimed in claim 1, wherein said chemotherapy further comprises the administration of an additional anti-cancer agent.

8. A method as claimed in claim 7, wherein said additional anti-cancer agent is a topoisomerase inhibitor.

9. A method as claimed in any preceding claim, wherein said biological sample is fixed, paraffin-embedded, or fresh, or frozen.

10. A method as claimed in any preceding claim, wherein said RNA is isolated from a fixed, paraffin-embedded breast cancer tissue specimen of said subject.

11. A method as claimed in any preceding claim, wherein the beneficial response is a clinical complete response.

12. A method as claimed in any preceding claim, wherein the beneficial response is a pathological complete response.

13. A method as claimed in any preceding claim, wherein the expression level of said RNA transcript is determined by reverse transcription-polymerase chain reaction (RT-PCR).

14. A method as claimed in any preceding claim, further comprising determining the level of an RNA transcript of GRB7, HER2, EstR1, PR, Bcl2, CEGP1, SURV, Ki67, MYBL2, CCNB1, STK15, CTSL2, STMY3, CD68, GSTM1, and BAG1, or their expression products.

**Patentansprüche**

1. Verfahren zur Bestimmung, ob ein Spiegel eines RNA-Transkripts mit einem vorteilhaften Ansprechen auf eine ein Anthracyclin und ein Taxan umfassende Chemotherapiebehandlung assoziiert ist, wobei man bei dem Verfahren:

   einen Spiegel eines RNA-Transkripts in einer Brustkrebszellen umfassenden biologischen Probe bestimmt, die vor einer Chemotherapie von einem mit Brustkrebs diagnostizierten menschlichen Individuum erhalten wurde, wobei es sich bei dem RNA-Transkript um das RNA-Transkript von BAG1 handelt; den Spiegel des RNA-Transkripts von BAG1 gegen einen Spiegel wenigstens einer Referenz-RNA normiert, so dass man ein normiertes BAG1-Expressionsniveau erhält;
   das normierte BAG1-Expressionsniveau von dem Individuum mit einem normierten BAG1-Expressionsniveau in Brustkrebspatienten-Referenzproben vergleicht, die vor einer Chemotherapie von mit einer ein Anthracyclin und ein Taxan umfassenden Chemotherapie behandelten Brustkrebspatienten erhalten wurden; und
   bestimmt, dass das normierte BAG1-Expressions-niveau von dem Individuum mit einer verminderten Wahrscheinlichkeit eines vorteilhaften Ansprechens auf eine ein Anthracyclin und ein Taxan umfassende Chemotherapiebehandlung assoziiert ist, wenn das normierte BAG1-Expressionsniveau von dem Individuum verglichen mit dem normierten BAG1-Expressionsniveau in den von Patienten, die ein vorteilhaftes Ansprechen auf eine ein Anthracyclin und ein Taxan umfassende Chemotherapiebehandlung zeigten, erhaltenen Brustkrebspatienten-Referenzproben erhöht ist.

2. Verfahren nach Anspruch 1, wobei es sich bei der Chemotherapie um Adjuvans-Chemotherapie oder Neo-adjuvans-Chemotherapie handelt.

3. Verfahren nach Anspruch 1, wobei es sich bei dem Taxan um Docetaxel oder Paclitaxel handelt.

4. Verfahren nach Anspruch 1, wobei es sich bei dem Anthracyclin um Doxorubicin handelt.

5. Verfahren nach Anspruch 1, wobei es sich bei dem Brustkrebs um einen invasiven Brustkrebs handelt.

6. Verfahren nach Anspruch 1, wobei es sich bei dem Brustkrebs um einen Brustkrebs der Stufe II oder Stufe III handelt.

7. Verfahren nach Anspruch 1, wobei die Chemotherapie ferner die Verabreichung eines zusätzlichen Anti-krebsmittels umfasst.

8. Verfahren nach Anspruch 7, wobei es sich bei dem zusätzlichen Antikrebsmittel um einen Topoisomerase-Inhibitor handelt.

9. Verfahren nach einem vorhergehenden Anspruch, wobei die biologische Probe fixiert, in Paraffin eingebettet oder frisch oder gefroren ist.

10. Verfahren nach einem vorhergehenden Anspruch, wobei die RNA von einem fixierten, in Paraffin eingebetteten Brustkrebsgewebepräparat des Individuums isoliert wird.

11. Verfahren nach einem vorhergehenden Anspruch, wobei es sich bei dem vorteilhaften Ansprechen um ein klinisches vollständiges Ansprechen handelt.

12. Verfahren nach einem vorhergehenden Anspruch, wobei es sich bei dem vorteilhaften Ansprechen um ein pathologisches vollständiges Ansprechen handelt.

13. Verfahren nach einem vorhergehenden Anspruch, wobei das Expressionsniveau des RNA-Transkripts mit Reverse-Transkription-Polymerasekettenreaktion (RT-PCR) bestimmt wird.

14. Verfahren nach einem vorhergehenden Anspruch, bei dem man ferner den Spiegel eines RNA-Transkripts von GRB7, HER2, EstR1, PR, Bcl2, CEGP1, SURV, Ki.67, MYBL2, CCNB1, STK15, CTSL2, STMY3, CD68, GSTM1 und BAG1 oder ihrer Expressionsprodukte bestimmt.

## Revendications

1. Procédé pour déterminer si un taux d'un produit de transcription d'ARN, est associé à une réponse bénéfique à un traitement chimiothérapeutique comprenant une anthracycline et un taxane, le procédé comprenant :

   la détermination d'un taux d'un produit de transcription d'ARN dans un échantillon biologique comprenant des cellules de cancer du sein obtenues avant chimiothérapie à partir d'un sujet humain diagnostiqué avec un cancer du sein, le produit de transcription d'ARN étant le produit de transcription d'ARN de BAG1 ;
   la normalisation du taux du produit de transcription d'ARN de BAG1 par rapport au taux d'au moins un ARN de référence pour obtenir un taux d'expression normalisé de BAG1 ;
   la comparaison du taux d'expression normalisé de BAG1 du sujet à un taux d'expression normalisé de BAG1 dans des échantillons de patients atteints de cancer du sang de référence obtenus avant chimiothérapie à partir de patients atteints de cancer du sein traités avec une chimiothérapie comprenant une anthracycline et un taxane ; et
   la détermination du fait que le taux d'expression normalisé de BAG1 du sujet est ou non associé à une probabilité réduite d'une réponse bénéfique à un traitement chimiothérapeutique comprenant une anthracycline et un taxane si le taux d'expression normalisé de BAG1 du sujet est augmenté par rapport au taux d'expression normalisé de BAG1 dans les échantillons de patients atteints de cancer du sein obtenu à partir de patients qui présentent une réponse bénéfique à un traitement chimiothérapeutique comprenant une anthracycline et un taxane.

2. Procédé selon la revendication 1, dans lequel ladite chimiothérapie est une chimiothérapie adjuvante ou une chimiothérapie néoadjuvante.

3. Procédé selon la revendication 1, dans lequel ledit taxane est le docétaxel ou le paclitaxel.

4. Procédé selon la revendication 1, dans lequel ladite anthracycline est la doxorubicine.

5. Procédé selon la revendication 1, dans lequel ledit cancer du sein est un cancer du sein invasif.

6. Procédé selon la revendication 1, dans lequel ledit cancer du sein est un cancer du sein de stade II ou de stade III.

**7.** Procédé selon la revendication 1, dans lequel ladite chimiothérapie comprend en outre l'administration d'un agent anticancéreux additionnel.

**8.** Procédé selon la revendication 7, dans lequel ledit agent anticancéreux additionnel est un inhibiteur de topoisomérase.

**9.** Procédé selon l'une quelconque des revendications précédentes, dans lequel ledit échantillon biologique est fixé, inclus dans la paraffine, ou frais, ou congelé.

**10.** Procédé selon l'une quelconque des revendications précédentes dans lequel ledit ARN est isolé à partir d'un spécimen de tissu de cancer du sein dudit sujet fixé, inclus dans la paraffine.

**11.** Procédé selon l'une quelconque des revendications précédentes, dans lequel la réponse bénéfique est une réponse clinique totale.

**12.** Procédé selon l'une quelconque des revendications précédentes, dans lequel la réponse bénéfique est une réponse pathologique totale.

**13.** Procédé selon l'une quelconque des revendications précédentes, dans lequel le taux d'expression dudit produit de transcription d'ARN est déterminé par réaction de polymérase en chaîne par transcription inverse (RT-PCR).

**14.** Procédé selon l'une quelconque des revendications précédentes, comprenant en outre la détermination du taux d'un produit de transcription d'ARN de GRB7, HER2, EstR1, PR, Bcl2, CEGP1, SURV, Ki.67, MYBL2, CCNB1, STK15, CTSL2, STMY3, CD68, GSTM1, et BAG1, ou leurs produits d'expression.

FIGURE 1

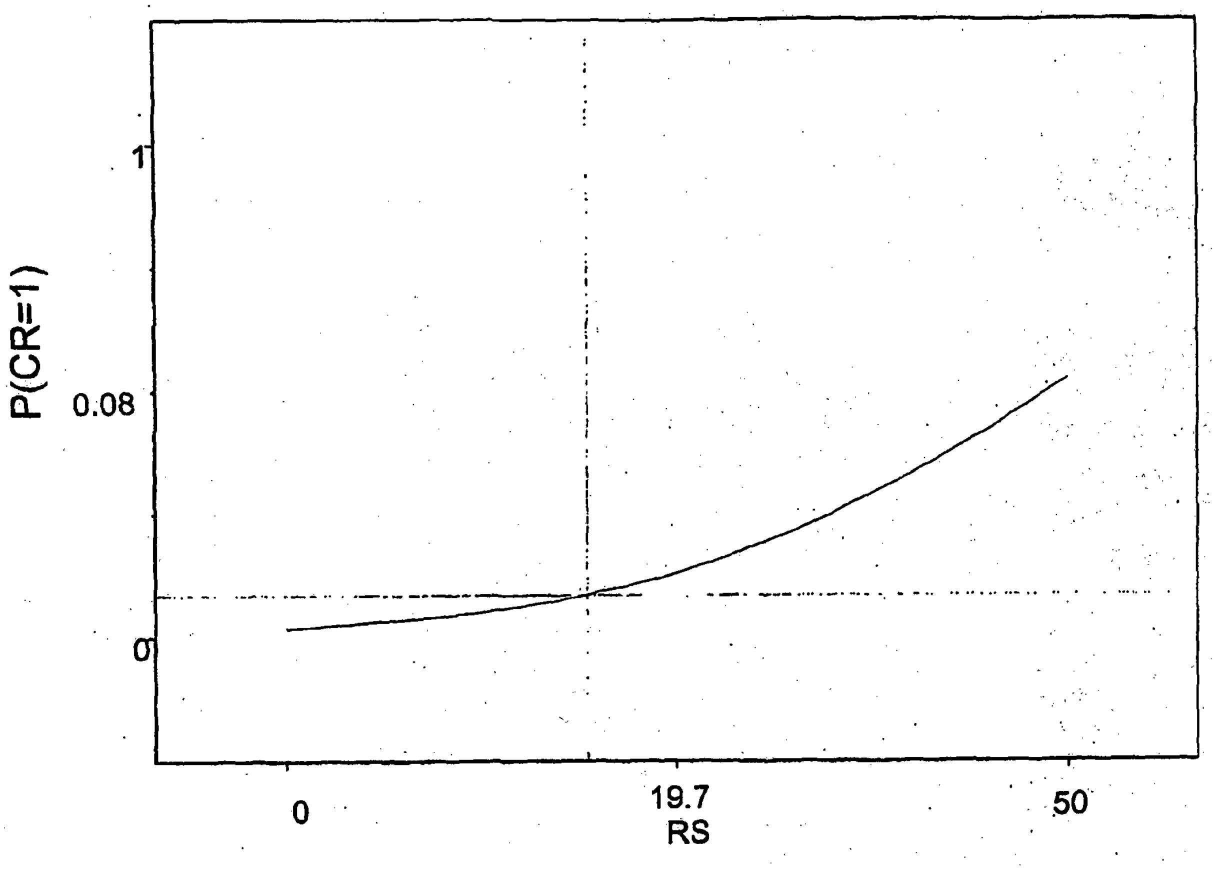

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004111603 A
- US 60486302 B
- US 486302 P

### Non-patent literature cited in the description


- **GOLUB et al.** *Science,* 1999, vol. 286, 531-537
- **BHATTACHARJAE et al.** *Proc. Natl. Acad. Sci. USA,* 2001, vol. 98, 13790-13795
- **CHEN-HSIANG et al.** *Bioinformatics,* 2001, vol. 17 (1), S316-S322
- **RAMASWAMY et al.** *Proc. Natl. Acad. Sci. USA,* 2001, vol. 98, 15149-15154
- **MARTIN et al.** *Cancer Res.,* 2000, vol. 60, 2232-2238
- **WEST et al.** *Proc. Natl. Acad. Sci. USA,* 2001, vol. 98, 11462-11467
- **SORLIE et al.** *Proc. Natl. Acad Sci. USA,* 2001, vol. 98, 10869-10874
- **YAN et al.** *Cancer Res.,* 2001, vol. 61, 8375-8380
- **PEROU et al.** *Nature,* 2000, vol. 406, 747-752
- **DING et al.** *Proceedings of the American Association for Cancer Research,* 2000, vol. 41, 404
- **KITADA et al.** *Blood,* 1998, vol. 91, 3379-89
- **MUSS et al.** *N. Eng. J. Med.,* 1994, vol. 330, 1260-6
- **SJÖSTRÖM et al.** *Clin. Cancer Res.,* 2002, vol. 8, 811-6
- **SINGLETON et al.** Dictionary of Microbiology and Molecular Biology. J. Wiley & Sons, 1994
- **MARCH.** Advanced Organic Chemistry Reactions, Mechanisms and Structure. John Wiley & Sons, 1992
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **SAMBROOK et al.** Molecular Clowning: A Laboratory Manual. 1989
- Oligonucleotide Synthesis. 1984
- Animal Cell Culture. 1987
- Methods in Enzymology. Academic Press, Inc, 1987
- Handbook of Experimental Immunology. Blackwell Science Inc,
- Gene Transfer Vectors for Mammalian Cells. 1987
- Current Protocols in Molecular Biology. 1987
- PCR: The Polymerase Chain Reaction. 1994
- **PARKER ; BARNES.** *Methods in Molecular Biology,* 1999, vol. 106, 247-283
- **HOD.** *Biotechniques,* 1992, vol. 13, 852-854
- **WEIS et al.** *Trends in Genetics,* 1992, vol. 8, 263-264
- **AUSUBEL et al.** Current Protocols of Molecular Biology. John Wiley and Sons, 1997
- **RUPP ; LOCKER.** *Lab Invest.,* 1987, vol. 56, A67
- **DE ANDRESS et al.** *BioTechniques,* 1995, vol. 18, 42044
- **HELD et al.** *Genome Research,* 1996, vol. 6, 986-994
- **DING ; CANTOR.** *Proc. Natl. Acad Sci. USA,* 2003, vol. 100, 3059-3064
- **LIANG ; PARDEE.** *Science,* 1992, vol. 257, 967-971
- **KAWAMOTO et al.** *Genome Res.,* 1999, vol. 12, 1305-1312
- **OLIPHANT et al.** *Discovery of Markers for Disease,* June 2002
- **FERGUSON et al.** *Analytical Chemistry,* 2000, vol. 72, 5618
- **YANG et al.** *Genome Res.,* 2001, vol. 11, 1888-1898
- **FUKUMURA et al.** *Nucl. Acids. Res.,* 2003, vol. 31 (16), e94
- **SCHENA et al.** *Proc. Natl. Acad. Sci. USA,* 1996, vol. 93 (2), 106-149
- **VELCULESCU et al.** *Science,* 1995, vol. 270, 484-487
- **VELCULESCU et al.** *Cell,* 1997, vol. 88, 243-51
- **BRENNER et al.** *Nature Biotechnology,* 2000, vol. 18, 630-634
- **T.E. GODFREY et al.** *J. Molec. Diagnostics,* 2000, vol. 2, 84-91
- **K. SPECHT et al.** *Am. J. Pathol.,* 2001, vol. 158, 419-29
- **FISHER et al.** *J. Clin. Oncology,* 1997, vol. 15, 2002-2004

- **FISHER et al.** *J. Clin. Oncology,* 1998, vol. 16, 2672-2685
- **VAN DER HAGE et al.** *J. Clin. Oncol.,* 2001, vol. 19, 4224-4237
- **HOLMES et al.** *J. Natl. Cancer Inst.,* 1991, vol. 83, 1797-1805
- **MOLITERNI et al.** *Seminars in Oncology,* 1999, vol. 24, S17-10, S-17-14
- **CLEATOR et al.** *Endocrine-Related Cancer,* 2002, vol. 9, 183-195
- User Bulletin #2'' for the ABI PRISM 7700 Sequence Detection System. *Applied Biosystems,* 1997
- **VAN K. BOROOAH.** Ordered Multinominal Models. *LOGIT and PROBIT,* 2002